# EUROPEAN PATENT APPLICATION

(11) **EP 3 964 568 A1**
(43) Date of publication of application: **09.03.2022**
(21) Application number: 20782974.8
(22) Date of filing: 03.04.2020
(51) Int. Cl.: C12N 5/10, C12N 15/12, A61P 35/00

(54) **IMMUNE CELL CONTAINING TUMOR ANTIGEN RECOGNITION RECEPTOR AND APPLICATION THEREOF**

(30) Priority: 04.04.2019 CN 201910271566
(71) Applicant: Shanghai Pharmaceuticals Holding Co., Ltd., Shanghai 201203 (CN)
(72) Inventor: LIAO, Xuemei, Shanghai 201203 (CN); HUANG, Jingwei, Shanghai 201203 (CN); CHEN, Zhuo, Shanghai 201203 (CN); XIE, Manman, Shanghai 201203 (CN); LI, Andi, Shanghai 201203 (CN); LIU, Yang, Shanghai 201203 (CN); XIA, Guangxin, Shanghai 201203 (CN); LIU, Yanjun, Shanghai 201203 (CN); KE, Ying, Shanghai 201203 (CN)
(74) Representative: Böhm, Brigitte
(86) International application number: PCT/CN2020/083129
(87) International publication number: WO 2020/200303

(57) **Abstract**

A modified immune effector cell and a use thereof, a cell population containing the immune effector cell, and a pharmaceutical composition. The expression and/or activity of an S1PR1 protein of the modified immune effector cell is up-regulated.

## Description

### TECHNICAL FIELD

The present application relates to the field of biomedicines, and in particular, relates to a modified immune effector cell and uses thereof, particularly an immune effector cell in which the expression and/or activity of an S1PR1 protein is up-regulated.

### BACKGROUND ART

In the CAR-T (or TCR-T) therapy, memory CAR-T cells are the same as memory T cells, and more tend to home to lymph nodes (https://doi.org/10.1124/jpet.118.252858). However, since the activation of the CAR-T cells depends on the direct recognition of a target cell membrane antigen by an antigen recognition region, the memory CAR-T cells that home to the lymph nodes usually cannot be effectively activated. However, if the memory CAR-T cells reside in the lymph nodes for a long time without contacting target cells, the efficacy will be affected.

Central memory T lymphocytes more tend to home to the lymph nodes in the peripheral circulation due to their expressions of for example homing receptors CCR7 and adhesion molecule receptors CD62L. After entering into the lymph nodes and if activated by antigen-presenting cells (such as DC cells), the central memory T lymphocytes will proliferate in quantity and differentiate to form effector cells to reduce the expression of homing receptors, and meanwhile up-regulate the expression of for example S1PR1 (sphingosine-1-phosphate receptor 1) to migrate out of the lymph nodes, thereby reaching a treatment site. The CAR-T cells, wholes activation condition does not depend on the antigen-presenting cells, are activated after binding to target cells. Therefore, it is difficult to activate the CAR-T cells and migrate them out of the lymph nodes when there is no target cell in the lymph nodes. Consequently, the CAR-T cells reside in the lymph nodes and fail to function.

Nevertheless, there is no record in the prior art on how to promote the outward migration of the CAR-T (or TCR-T) cells from the lymph nodes.

### SUMMARY OF THE INVENTION

The present application provides a modified immune effector cell and uses thereof, wherein the expression and/or activity of an S1PR1 protein of the modified immune effector cell is up-regulated. The immune effector cell of the present application is more capable of migrating from a lymph node to more easily reach a treatment site for fulfilling a function, and has an enhanced immune effector function to further achieve an enhanced anti-tumor ability. The present application further provides a cell population containing the immune effector cell and a pharmaceutical composition.

In one aspect, the present application provides a modified immune effector cell, in which the expression and/or activity of an S1PR1 protein is up-regulated compared with an unmodified immune effector cell.

In some embodiments, the immune effector cell comprises a T cell, a lymphocyte, a granulocyte and/or a peripheral blood mononuclear cell.

In some embodiments, the immune effector cell comprises a memory T cell.

In some embodiments, in the modified immune cell, the expression and/or activity of a nucleic acid molecule encoding the S1PR1 protein is up-regulated.

In some embodiments, the modified immune cell additionally contains the S1PR1 protein.

In some embodiments, the modified immune effector cell additionally contains the nucleic acid molecule encoding the S1PR1 protein.

In some embodiments, the modified immune effector cell contains a vector, which comprises the nucleic acid molecule encoding the S1PR1 protein.

In some embodiments, the immune effector cell expresses a chimeric antigen receptor (CAR).

In some embodiments, the modified immune effector cell contains a vector, which includes a nucleic acid molecule encoding the chimeric antigen receptor.

In some embodiments, the nucleic acid molecule encoding the chimeric antigen receptor and the nucleic acid molecule encoding the S1PR1 protein are located in the same vector.

In some embodiments, the nucleic acid molecule encoding the chimeric antigen receptor is located at a 5'-terminus of the nucleic acid molecule encoding the S1PR1 protein; or, the nucleic acid molecule encoding the chimeric antigen receptor is located at a 3'-terminus of the nucleic acid molecule encoding the S1PR1 protein.

In some embodiments, the chimeric antigen receptor comprises a chimeric antigen receptor targeting a tumor-specific antigen, wherein the tumor-specific antigen is selected from the group consisting of: EpCAM, Mesothelin (MSLN), CEA, IL13, PDPN, VEGF, EGFR, EGFRvIII, PSMA, FAP, CD171, GD2, Glypican-2, Glypican-3, HER2, HPV antigen, cyclin D1, p53, MMP-7, IL13Ralpha2, MMP-2, MUC-1, G250, L1CAM, ROR1, and GPC3.

In some embodiments, the chimeric antigen receptor contains an antigen-binding domain that specifically binds to the tumor-specific antigen.

In some embodiments, the antigen-binding domain comprises a single-chain antibody.

In some embodiments, the antigen-binding domain comprises a single-chain antibody targeting GPC3 or MSLN.

In some embodiments, the single-chain antibody includes an amino acid sequence as set forth in any of SEQ ID NOs: 1 to 2.

In some embodiments, the chimeric antigen receptor contains a transmembrane domain.

In some embodiments, the transmembrane domain includes a transmembrane domain derived from a protein selected from the group consisting of: CD3ζ, CD28, 4-1BB, OX40, SLAMF4, CD127, NKG2D, ICOS, and FcγRIIIa.

In some embodiments, the chimeric antigen receptor contains a costimulatory domain.

In some embodiments, the costimulatory domain includes a costimulatory domain selected from the following proteins or a combination thereof: CD137, CD28, OX40, ICOS, DAP10, 2B4, CD27, CD30, CD40, CD40L, TIM1, CD226, DR3, SLAM, NKG2D, CD244, FceRIy, BTLA, GITR, HVEM, CD2, NKG2C, LIGHT, and DAP12.

In some embodiments, the chimeric antigen receptor contains a hinge region, which connects the antigen-binding domain and the transmembrane domain.

In some embodiments, the hinge region includes a hinge region derived from a protein selected from the group consisting of: CD8, CD28, IgG, 4-1BB, CD4, CD27, CD7, PD-1, and CH2CH3.

In some embodiments, the chimeric antigen receptor contains an amino acid sequence set forth in any of SEQ ID NOs: 5 to 6.

In some embodiments, the immune effector cell comprises a nucleic acid molecule encoding the chimeric antigen receptor.

In some embodiments, a vector containing the nucleic acid molecule encoding the chimeric antigen receptor and the nucleic acid molecule encoding the S1PR1 protein contains a nucleotide sequence set forth in any one of SEQ ID NOs: 7 to 9.

In another aspect, the present application provides a cell population containing the immune effector cell.

In another aspect, the present application provides a pharmaceutical composition containing the immune effector cell and/or the cell population, and optionally a pharmaceutically acceptable carrier.

In another aspect, the present application provides a use of the immune effector cell, the cell population and/or the pharmaceutical composition in the preparation of a drug for the treatment of a tumor.

In some embodiments, the tumor comprises a solid tumor.

In some embodiments, the tumor comprises pancreatic cancer, glioma, liver cancer and/or colon cancer.

Those skilled in the art can easily perceive other aspects and advantages of the present application from the detailed description below. The detailed description below only shows and describes exemplary embodiments of the present application. As will be appreciated by those skilled in the art, the content of the present application enables those skilled in the art to make changes to the disclosed specific embodiments without departing from the spirit and scope of the invention involved in the present application. Correspondingly, the accompanying drawings and the descriptions in the specification of the present application are merely for an exemplary rather than restrictive purpose.

### BRIEF DESCRIPTION OF THE DRAWINGS

The specific features of the invention involved in the present application are listed in the appended claims. The characteristics and advantages of the invention involved in the present application can be better understood by referring to the exemplary embodiments and the accompanying drawings described in detail below. The accompanying drawings are briefly illustrated as follows:
FIG. 1 shows a positive rate of CAR T cells of the present application;
FIG. 2 shows the flow cytometry results of GFP-positive CAR T cells of the present application in a lower chamber at a gradient concentration SIP in a migration experiment;
FIG. 3 shows the GFP-positive rate of the CAR T cells of the present application in the lower chamber at the gradient concentration SIP in the migration experiment;
FIG. 4 shows the migration efficiency of the CAR T cells of the present application in the lower chamber in the migration experiment;
FIG. 5 shows the killing efficiency of the CAR T cells of the present application on target cells;
FIG. 6 shows the killing efficiency of the CAR T cells of the present application on target cells;
FIG. 7 shows the killing efficiency of the CAR T cells of the present application on target cells;
FIG. 8 shows that the CAR T cells of the present application secrete cytokines under the stimulation of target cells, with A indicating IL-2 and B indicating IFN-γ;
FIG. 9 shows that the CAR T cells of the present application inhibit tumor growth in vivo, with A indicating tail vein administration and B indicating intratumoral injection administration;
FIG. 10 shows the content of T cells in peripheral blood on Day 28 in an in-vivo migration experiment;
FIG. 11 shows that the CAR T cells of the present application inhibit tumor growth in vivo; and
FIG. 12 shows the relationship between a content of CD3⁺T cells in peripheral blood and a tumor volume, with A indicating GC33CAR and B indicating GC33CAR-IRES-S1PR1.

### DETAILED DESCRIPTION OF THE PREFERRED EMBODIMENTS

The embodiments of the invention of the present application will be illustrated by specific examples below. Those familiar with this technology can easily understand other advantages and effects of the invention of the present application from the content disclosed in the specification.

### TERMS & DEFINITIONS

In the present application, the term "S1PR1" generally refers to sphingosine-1-phosphate receptor 1, also known as endothelial differentiation gene 1 (EDG1). S1PR1 is a G protein-coupled receptor, which binds to a biologically active signal molecule sphingosine 1-phosphate (SIP). This term may include natural S1PR1 or variants thereof, and synthetic S1PR1. The S1PR1 may also comprise full-length S 1PR1, or truncated forms, spliced forms or functional fragments of S1PR1. In some cases, S1PR1 in the present application may be full-length human S1PR1; a nucleotide sequence of the full-length human S1PR1 gene can be found under NCBI Accession No. NM_001400; and an amino acid sequence of the S1PR1 protein can be found under NCBI Accession No. NP_001391. In the present application, the terms "MSLN", "Mesothelin" and "Mesothelins" can be used interchangeably and generally refer to a glycoprotein on the surface of a cell (for example, a tumor cell) linked to glycosyl phosphatidyl inositol (GPI). The term "MSLN" may include a mature MSLN protein, various subtypes of the MSLN protein and precursor proteins thereof, as well as a natural MSLN protein or variants, spliced forms and truncated forms thereof, or synthetic MSLN proteins. The MSLN precursor protein may include a mesothelin subtype 1 precursor protein and a mesothelin subtype 2 precursor protein, and these precursor proteins may be processed (for example, by enzymatic digestion) to generate the mature MSLN. MSLN may be linked to a megakaryocyte-potentiating factor (MPF). An amino acid sequence of the mesothelin subtype 1 precursor protein can be found under NCBI Accession No. NP_001170826 or NP_005814, and an amino acid sequence of the mesothelin subtype 2 precursor protein can be found under NCBI Accession No. NP_037536.

In the present application, the term "GPC3" is also referred to as glypican-3, which generally refers to a complex formed by covalently linking proteins, sugars and lipids, and can be expressed on a cell surface by glycosyl phosphatidyl inositol (GPI). This term may include various subtypes (for example, a subtype 1, a subtype 2, a subtype 3, and a subtype 4), variants, precursors, and spliced forms of natural GPC3, as well as synthetic GPC3 or functions fragments thereof. For example, an amino acid sequence of a precursor form of the GPC3 subtype 1 can be found under NCBI Accession No. NP_001158089; an amino acid sequence of a precursor form of the GPC3 subtype 3 may be found under NCBI Accession No. NP_001158090; an amino acid sequence of a precursor form of the GPC3 subtype 4 can be found under NCBI Accession No. NP_001158091; and an amino acid sequence of a precursor form of the GPC3 subtype 2 can be found under NCBI Accession No. NP_004475.

In the present application, the term "chimeric antigen receptor (CAR)" generally refers to a fusion protein containing an extracellular domain capable of binding to an antigen and at least one intracellular domain. CAR is a core component of a chimeric antigen receptor T cell (CAR-T), and may include an antigen (for example, a tumor-specific antigen and/or a tumor-associated antigen) binding domain, a transmembrane domain, a costimulatory domain, and an intracellular signal domain. In the present application, CAR may be combined with an activated intracellular domain of the T cell receptor based on the antigen (for example, CD70) specificity of the antibody. Genetically modified T cells expressing CAR may specifically recognize and eliminate malignant cells expressing target antigens. For the descriptions of CAR and CAR-T cells, references may be made to, for example, Sadelain M, Brentjens R, Rivi 'ere I. The basicprinciples of chimeric antigen receptor design. Cancer Discov. 2013;3(4):388-398; Turtle CJ, Hudecek M, Jensen MC, Riddell SR. Engineered T cells for anti-cancer therapy. Curr Opin Immunol. 2012;24(5):633-639; Dotti G,Gottschalk S, Savoldo B, Brenner MK. Design and development of therapies using chimeric antigen receptor-expressing T cells. Immunol Rev. 2014;257(1): 107-126; and WO2013154760, WO2016014789.

In the present application, the term "antigen-binding domain" generally refers to a domain capable of binding to a target antigen. The antigen-binding domain may contain a chimeric antigen receptor and a fragment thereof, or an antibody or an antigen-binding fragment thereof, which can specifically bind to an antigen. The antigen-binding domain may be of a natural source, a synthetic source, a semi-synthetic source, or a recombinant source. In some cases, the antigen-binding domain may include a single-chain antibody.

In the present application, the term "antibody" generally refers to a polypeptide molecule that can specifically recognize and/or neutralize a specific antigen. For example, the antibody may contain an immunoglobulin consisting of at least two heavy (H) chains and two light (L) chains that are interconnected by disulfide bonds, and may include any molecule containing an antigen-binding portion thereof. The term "antibody" comprises monoclonal antibodies, antibody fragments or antibody derivatives, including but not limited to human antibodies, humanized antibodies, chimeric antibodies, single domain antibodies (for example, dAb), single-chain antibodies (for example, scFv), and antibody fragments (for example, Fab, Fab' and (Fab) 2 fragments) that bind to the antigen. The term "antibody" also comprises all recombinant forms of antibodies, such as antibodies expressed in prokaryotic cells, unglycosylated antibodies, and any antigen-binding antibody fragments and derivatives thereof as described in the present application. Each heavy chain may consist of a heavy-chain variable region (VH) and a heavy-chain constant region. Each light chain may consist of a light-chain variable region (VL) and a light-chain constant region. The VH and VL regions may be further distinguished as hypervariable regions that are called complementarity determining regions (CDR), which are interspersed in more conserved regions that are called framework regions (FR). Each VH or VL may consist of three CDRs and four FR regions, which may be arranged in the following order from an amino terminus to a carboxy terminus: FR1, CDR1, FR2, CDR2, FR3, CDR3, and FR4. The heavy-chain and light-chain variable regions contain binding domains that interact with antigens.

In the present application, the term "single-chain antibody (scFv)" may be an antibody formed by linking a heavy-chain variable region and a light-chain variable region of the antibody by a linker. In the present application, the term "transmembrane domain" generally refers to a domain in the CAR that crosses a cell membrane, which is linked to an intracellular signal transduction domain and plays a role in transmitting signals.

In the present application, the term "costimulatory domain" generally refers to an intracellular domain that may provide an immune costimulatory molecule, which is a cell surface molecule required by lymphocytes for an effective response to an antigen.

In the present application, the term "hinge region" generally refers to a linker region between the antigen-binding domain and the transmembrane region.

In the present application, the term "signaling domain" generally refers to an intracellular domain capable of transducing signals. In the present application, the intracellular signaling domain may transmit signals into a cell. Generally, the signaling domain is any continuous amino acid sequence for directing protein targeting. For example, the intracellular signaling domain is an intracellular signaling domain of the chimeric antigen receptor.

In the present application, the term "immune effector cell" generally refers to an immune cell that participates in an immune response and exercises an effector function. For example, said exercising the effector function may include removing a foreign antigen, promoting an immune effector response, etc. In some cases, the immune effector cell may be stimulated by an antigen on the surface of a target cell to produce an immune response, for example, to secrete cytokines. Examples of the immune cell include but not limited to: plasma cells, T cells, B cells, natural killer (NK) cells, natural killer T (NKT) cells, mast cells, granulocytes, monocytes, lymphocytes, dendritic cells, and macrophages. The term also includes engineered immune cells, such as immune cells that have been genetically modified by adding exogenous genetic materials in the form of DNA or RNA to total genetic materials of the cells.

In the present application, the term "T cell receptor (TCR)" generally refers to a specific receptor on the surface of a T cell. The T cell receptor is a heterodimer and may consist of two different subunits. Most of the T cell receptors (for example, 95% and above, 96% and above, 97% and above, etc.) consist of α subunits and β subunits, and each peptide chain may be divided into portions including a variable region (V region), a constant region (C region), a transmembrane region, and a cytoplasmic region. In the present application, the terms "polypeptide", "peptide", and "protein" can be used interchangeably and generally refer to polymers having amino acids of any length. Theses polymers, either linear or branched, may contain modified amino acids, and may be interrupted by non-amino acids. These terms also cover amino acid polymers that have been modified. These modifications may include: formation of a disulfide bond, glycosylation, lipidation, acetylation, phosphorylation, or any other manipulations (such as binding to a labeled component). The term "amino acid" comprises natural and/or unnatural or synthetic amino acids (including glycine and D- and L-optical isomers), as well as amino acid analogs and peptide mimetics.

In the present application, the terms "polynucleotide", "nucleotide", "nucleotide sequence", "nucleic acid" and "oligonucleotides" can be used interchangeably and generally refer to a polymetric form of nucleosides of any length, such as deoxyribonucleotides or ribonucleotides, or analogs thereof. The polynucleotide may have any three-dimensional structure and may exercise any known or unknown function. The following are non-limiting examples of the polynucleotides: coding or noncoding regions of genes or gene fragments, multiple loci (one locus) defined by linkage analysis, exons, introns, messenger RNA (mRNA), transfer RNA, ribosomal RNA, short interfering RNA (siRNA), short hairpin RNA (shRNA), micro-RNA (miRNA), ribozyme, cDNA, recombinant polynucleotide, branched polynucleotide, plasmid, vector, isolated DNA of any sequence, isolated RNA of any sequence, nucleic acid probes, and primers. The polynucleotide may contain one or more modified nucleotides, such as methylated nucleotides and nucleotide analogs. If any, a nucleotide structure may be modified before or after the assembly of a polymer. A nucleotide sequence may be interrupted by a non-nucleotide component. The polynucleotides may be further modified for example by conjugation with labeled components after polymerization.

In the present application, the "vector" generally refers to a nucleic acid molecule capable of self-replication in a suitable host, and is used to transfer an inserted nucleic acid molecule into and/or between host cells. The vector may include a vector mainly for inserting DNA or RNA into cells, a vector mainly for replicating DNA or RNA, and a vector mainly for expressing DNA or RNA transcription and/or translation. The vector also includes a carrier having a variety of the functions defined above. The vector may be a polynucleotide that may be transcribed and translated into a polypeptide when introduced into a suitable host cell. Generally, the vector may produce a desired expression product by culturing a suitable host cell containing the vector.

In the present application, the term "lentiviral vector" generally refers to an RNA viral vector. The lentiviral vector is a vector prepared based on the genome of a lentivirus by deleting many sequence structures related to the viral activity from the genome to achieve biological safety, and then introducing the sequence and expression structure of a target gene required by the experiment into a genome skeleton. Through the transfection via the lentiviral vector, the autologous genome and the carried exogenous genes can be randomly and stably integrated into the genome of the host cell. For example, the CAR molecule can be integrated into the host cell.

In the present application, the term "treatment" generally refers to the administration of one or more therapies (for example, one or more therapeutic agents, such as the nucleic acid molecule and/or immune effector cell according to the present application) to slow down or ameliorate the progression, severity and/or duration of proliferative disorders, or to ameliorate one or more symptoms (for example, one or more distinguishable symptoms) of the proliferative disorder. In the present application, the term "treatment" may also refer to the amelioration of at least one measurable physical parameter (such as tumor growth) of the proliferative disorder, regardless of whether it is discernible by a patient. The term "treatment" in the present application may also refer to the inhibition of the progression of the proliferative disorders in a physical manner by, for example, stabilizing discernible symptoms, or in a physiological manner by, for example, stabilizing physical parameters, or both. In some cases, the term "treatment" may refer to the reduction or stabilization of a tumor size or a cancer cell count.

In the present application, the term "and/or" should be understood to mean either or both of optional items.

In the present application, the term "containing" generally refers to the inclusion of explicitly specified features, but not excluding other elements.

In the present application, the term "approximately" generally refers to a variation within a range of 0.5%-10% above or below a specified value, for example, a variation within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, and 10% above or below a specified value.

### DETAILED DESCRIPTION OF THE INVENTION

The technical problem to be solved by the present application is to address the defect in the prior art that a CAR-T (or TCR-T) cell resides in a lymph node for a long time without contacting a target cell to consequently affect a therapeutic effect, and an immune cell containing a tumor antigen recognition receptor and uses thereof are provided. With overexpressed S1PR1 and the contained tumor antigen recognition receptor, the immune cell (for example, the CAR/TCR-T cell) according to the present application is more capable of migrating from the lymph node to more easily reach a treatment site for fulfilling a function, and has strong survivability.

The inventor of the present application creatively overexpresses S1PR1 in the immune cell (such as the memory CAR-T cell) while allowing the immune cell to contain the tumor antigen recognition receptor, which can preserve the memory of the CAR-T cell while avoiding the CAR-T cell residing inside the lymph node for a long time without contacting the target cell. As a result, the immune cell can more easily migrate from the lymph node to reach the treatment site. It was unexpectedly found that, when two proteins CAR and S1PR1 are linked, the overexpression of S1PR1 increases the survivability of the CAR-T cell. Compared with the prior art, the technical solution of the present application effectively enhances the memory formation and subsequent functions of the CAR-T cell, and consequently enhances its anti-tumor effect.

The present application mainly solves the above technical problems through the following technical solutions.

The present invention provides an immune cell containing a tumor antigen recognition receptor, where S1PR1 is overexpressed in the immune cell.

The tumor antigen recognition receptor may be a conventional tumor antigen recognition receptor in the art, for example, a chimeric antigen receptor (CAR) or a T cell receptor (TCR).

The CAR may be a conventional CAR in the art, and for example, may include an intracellular region, a hinge region, and a transmembrane region; and all the included intracellular region, hinge region, and transmembrane region may be conventional in the art. Examples of intracellular domains used in the present application include, but not limited to: a cytoplasmic portion of a surface receptor, costimulatory molecules, any molecules that act congruously to initiate signal transduction in the T cell, and any derivatives or variants of these elements and any synthetic sequences with the same functional capacity.

The intracellular domains described in the present application, for example, from TCR, CD3ζ, CD3γ, CD3δ, CD3ε, CD86, ordinary FcRy, FcRβ (FcεR1b), CD79a, CD79b, FcyRIIa, DAP10, DAP12, T cell receptor (TCR), CD8, CD27, CD28, 4-1BB (CD137), OX9, OX40, CD30, CD40, PD-1, ICOS, KIR family proteins, lymphocyte function associated antigen-1 (LFA-1), CD2, CD7, LIGHT, NKG2C, B7-H3, ligands specifically binding to CD83, CDS, ICAM-1, GITR, BAFFR, HVEM (LIGHTR), SLAMF7, NKp80 (KLRF1), CD127, CD160, CD19, CD4, CD8α, CD8β, IL2Rβ, IL2Ry, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46, NKG2D, Toll-like receptor1 (TLR1), TLR2, TLR3, TLR4, TLR5, TLR6, TLR7, TLR8, TLR9, or fragments or domains of other costimulatory molecules, can be combined with any antigen-binding domain described in the present application, any transmembrane domain described in the present application, or any other domains described in the present application that can be included in the CAR. For example, these intracellular domains may be derived from one or more of CD3ζ, CD28, 4-1BB, OX40, SLAMF4, CD127, NKG2D, ICOS, and FcγRIIIa.

For example, the intracellular region of the CAR may include a human 4-1BB intracellular region and/or a human CD28 intracellular region, and a human CD3ζ intracellular region. For example, the intracellular region of the CAR may include said human 4-1BB intracellular region, human CD28 intracellular region, and human CD3ζ intracellular region.

The antigens recognized by the CAR may be conventional antigens in the art, such as EpCAM, Mesothelin, CEA, IL13, PDPN, VEGF, EGFR, EGFRvIII, PSMA, FAP, CD171, GD2, Glypican-2, Glypican-3, HER2, HPV antigen, cyclin D1, p53, MMP-7, IL13Ralpha2, MMP-2, MUC-1, G250, L1CAM, ROR1, GPC3, or MSLN, etc. In some cases, the antigens recognized by the CAR described in the present application may be selected from the group consisting of GPC3, mesothelin, EGFRVIII, IL13Ra2, GPC, or CEA. For example, a CAR gene against the GPC3 may include a nucleotide sequence of SEQ ID NO: 3 as shown in the sequence list.

Here, the S1PR1 protein may include an amino acid sequence as set forth in NCBI Accession No. NP_001391, and the S1PR1 gene may include a nucleotide sequence as set forth in NCBI Accession No. NM_001400.

In the immune cell, the S1PR1 gene and the CAR or the TCR may be located on the same expression vector, or different expression vectors.

When the S1PR1 gene and the CAR or the TCR are located on the same vector, the expression vector may sequentially include a nucleotide sequence encoding S1PR1, IRES, and a nucleotide sequence encoding CAR; or may sequentially include the nucleotide sequence encoding S1PR1, IRES, and a nucleotide sequence encoding TCR; or may sequentially include the nucleotide sequence encoding S1PR1, 2A, and the nucleotide sequence encoding CAR; or may sequentially include the nucleotide sequence encoding S1PR1, 2A, and the nucleotide sequence encoding TCR; or may sequentially include the nucleotide sequence encoding CAR, IRES, and the nucleotide sequence encoding S1PR1; or may sequentially include the nucleotide sequence encoding TCR, IRES, and the nucleotide sequence encoding S1PR1; or may sequentially include the nucleotide sequence encoding CAR, 2A, and the nucleotide sequence encoding S1PR1; or may sequentially include the nucleotide sequence encoding TCR, 2A, and the nucleotide sequence encoding S1PR1. Here, the nucleotide sequence of IRES may be as shown in SEQ ID NO: 13 in the sequence list; the nucleotide sequence of 2A may be as shown in SEQ ID NO: 14 in the sequence list.

The immune cell described in the present application may be a T cell, for example, a memory T cell.

The present application further provides uses of the immune cell as defined above in the preparation of a drug for the treatment of a tumor, where the tumor may include pancreatic cancer, glioma, liver cancer, or colon cancer.

The present application further provides a pharmaceutical composition containing the immune cell as defined above.

The present application further provides a use of S1PR1 in the preparation of an immune cell containing a tumor antigen recognition receptor. For example, the tumor antigen recognition receptor may be a chimeric antigen receptor or a T cell receptor.

All the conditions above can be randomly combined following the common knowledge in the art to obtain all the examples of the present application.

The reagents and raw materials used in the present application are both commercially available. The present application has the following positive progressive effects:
With the overexpressed S1PR1 and the contained tumor antigen recognition receptor, the immune cell (for example, the CAR/TCR-T cell) according to the present application is more capable of migrating from the lymph node to more easily reach a treatment site for fulfilling a function; it can increase the in-vivo/vitro survivability of the immune cell containing the tumor antigen recognition receptor (for example, the CAR-T cell); and moreover, it is conducive to enhancing the memory formation and subsequent functions of the CAR-T cell to consequently enhance its anti-tumor effect.

### S1PR1

In one aspect, the present application provides a modified immune effector cell, in which the expression and/or activity of an S1PR1 protein is up-regulated compared with an unmodified immune effector cell. In some cases, the unmodified immune effector cell may not express the S1PR1 protein. In some cases, the unmodified immune effector cell may express the S1PR1 protein with a relatively low content to the extent that the function and effect of S1PR1 may not be exercised. For example, compared with the unmodified immune effector cell, the expression of the S1PR1 protein of the modified immune effector cells is up-regulated, and the expression of a protein related to a S1PR1 signal pathway is up-regulated. Protein quantification methods, such as a BCA quantitative method, a Bradford quantitative method, a Lowry detection method, and a spectrophotometric method, may be used to detect a protein expression level of cells. For example, compared with the unmodified immune effector cells, the activity of the S1PR1 protein of the modified immune effector cells is increased, which may be reflected in that the affinity of the modified immune effector cells to SIP is increased, and the activity of the S1PR1 signal pathway is up-regulated, and an immune effector function is enhanced.

For example, a reagent capable of up-regulating the expression and/or activity of the S1PR1 protein may be administered to the immune effector cell. The reagent may be a macromolecule (such as DNA, RNA, polypeptide or protein), a virus, a plasmid, an organic or inorganic micromolecule, or a combination of the above.

In some cases, the modified immune effector cell may additionally contain the S1PR1 protein. The immune effector cell may not contain the S1PR1 protein before modification, and after modification, the expression of the S1PR1 protein may be detected on the modified immune effector cell. In some cases, the S1PR1 in the modified immune effector cell may be directly overexpressed at a protein level. For example, the S1PR1 protein may be introduced into an immune effector cell that does not express S1PR1, to allow it express the S1PR1 protein. For example, the S1PR1 protein may be introduced into an immune effector cell that expresses the S1PR1 at a low level, to increase its expression level of the S1PR1 protein. For example, the S1PR1 gene may be introduced into an immune effector cell that does not express S1PR1, to allow it express the S1PR1 protein. For example, the S1PR1 gene may be introduced into an immune effector cell that expresses the S1PR1 at a low level, to increase its expression level of the S1PR1 protein.

In the present application, the expression and/or activity of the nucleic acid molecule encoding the S1PR1 protein in the modified immune effector cell is up-regulated compared with the unmodified immune effector cell. In some cases, the immune effector cell may not contain the nucleic acid molecule encoding the S1PR1 protein before modification; in other cases, the immune effector protein may contain but not express the nucleic acid molecule encoding the S1PR1 protein before modification; and in other cases, the immune effector protein may contain the nucleic acid molecule encoding the S1PR1 protein before modification, but at a low expression level.

In some cases, the modified immune effector cell additionally contains the nucleic acid molecule encoding the S1PR1 protein. For example, the nucleic acid molecule encoding the S1PR1 may be introduced into the immune effector cell (for example, by a vector, such as those described below), so that the modified immune cell additionally contains the nucleic acid molecule encoding the S1PR1 protein.

For example, a reagent may be administered to the immune effector cell, where the reagent may enhance the translation or transcription of the nucleic acid molecule encoding the S1PR1 protein. For example, the S1PR1 in the modified immune effector cell may be overexpressed on DNA and/or RNA. For example, the reagent may be a macromolecule (for example, DNA, RNA, polypeptide or protein), a virus, a plasmid, an organic or inorganic micromolecule, or a combination of the above.

For example, the S1PR1 gene may be introduced into the immune effector cell, so that the immune cell additionally contains the nucleic acid molecule encoding the S1PR1 protein. For example, the S1PR1 gene may be a nucleic acid molecule that contains and encodes a full-length S1PR1 protein, or a nucleic acid molecule that contains an encoding region of S1PR1. For example, the S1PR1 gene is constructed into a plasmid or viral vector, and then introduced into an immune cell.

The S1PR1 protein may be a full-length S1PR1 protein, or a truncated form, a spliced form or a functional fragment of the full-length S1PR1 protein. For example, the S1PR1 protein is a full-length human S1PR1 protein, and the nucleic acid molecule encoding the full-length human S1PR1 protein may include a nucleotide sequence shown in NCBI Accession No. NM_001400. CAR

In the present application, the immune cell described in the present application may include the chimeric antigen receptor (CAR) and/or the T cell receptor (TCR).

In the present application, the CAR may include a chimeric antigen receptor targeting a tumor-specific antigen, where the tumor-specific antigen may be selected from the group consisting of: EpCAM, Mesothelin (MSLN), CEA, IL13, PDPN, VEGF, EGFR, EGFRvIII, PSMA, FAP, CD171, GD2, Glypican-2, Glypican-3, HER2, HPV antigen, cyclin D1, p53, MMP-7, IL13Ralpha2, MMP-2, MUC-1, G250, L1CAM, ROR1, and GPC3.

In some cases, the CAR may contain an antigen-binding domain. The antigen-binding domain may bind to a tumor-specific antigen, where the tumor-specific antigen is selected from the group consisting of: EpCAM, Mesothelin (MSLN), CEA, IL13, PDPN, VEGF, EGFR, EGFRvIII, PSMA, FAP, CD171, GD2, Glypican-2, Glypican-3, HER2, HPV antigen, cyclin D1, p53, MMP-7, IL13Ralpha2, MMP-2, MUC-1, G250, L1CAM, ROR1, and GPC3. In some cases, the antigen-binding domain may include a single-chain antibody scFv. For example, the single-chain antibody may include a single-chain antibody GC33 targeting GPC3. For example, the single-chain antibody may include an amino acid sequence as set forth in SEQ ID NO: 1. For example, the single-chain antibody may include a single-chain antibody P4G2 targeting MSLN, for example, the single-chain antibody may include an amino acid sequence shown as set forth SEQ ID NO: 2.

In the present application, the CAR may contain a transmembrane domain. In some cases, the transmembrane domain may include a transmembrane domain derived from a protein selected from the group consisting of: CD3ζ, CD28, 4-1BB, OX40, SLAMF4, CD127, NKG2D, ICOS, and FcγRIIIa.

In the present application, the CAR may contain a costimulatory domain. In some cases, the costimulatory domain may include a costimulatory domain selected from the following proteins or a combination thereof: CD137, CD28, OX40, ICOS, DAP10, 2B4, CD27, CD30, CD40, CD40L, TIM1, CD226, DR3, SLAM, NKG2D, CD244, FceRIy, BTLA, GITR, HVEM, CD2, NKG2C, LIGHT, and DAP12.

In the present application, the CAR may contain a hinge region, which connects the antigen-binding domain and the transmembrane domain. In some cases, the hinge region may include a hinge region derived from a protein selected from the group consisting of: CD8, CD28, IgG, 4-1BB, CD4, CD27, CD7, PD-1, and CH2CH3.

In the present application, the CAR may include an antigen-binding domain, a hinge region, a transmembrane domain, a costimulatory domain, and/or a signaling domain. For example, the CAR may include an amino acid sequence as set forth in any one of SEQ ID NOs: 5 to 6.

The modified immune cell described in the present application may include the CAR. For example, the CAR may include an amino acid sequence as set forth in any one of SEQ ID Nos: 5 to 6.

The modified immune cell described in the present application may include a nucleic acid molecule encoding the CAR. For example, the nucleic acid molecule encoding the CAR may include a nucleotide sequence as set forth in any one of SEQ ID NOs: 3 to 4.

### Immune effector cell and cell population

In the present application, the modified immune effector cell may contain a vector, which may include the nucleic acid molecule encoding the S1PR1 protein. In the present application, the modified immune effector cell may contain a vector, which may include the nucleic acid molecule encoding the chimeric antigen receptor. In some cases, the nucleic acid molecule encoding the chimeric antigen receptor and the nucleic acid molecule encoding the S1PR1 protein may be located in the same vector. In other cases, the nucleic acid molecule encoding the chimeric antigen receptor and the nucleic acid molecule encoding the S1PR1 protein may be located in different vectors. When the nucleic acid molecule encoding the chimeric antigen receptor and the nucleic acid molecule encoding the S1PR1 protein are located in the same vector, in some cases, the nucleic acid molecule encoding the chimeric antigen receptor may be located at a 5'-terminus of the nucleic acid molecule encoding the S1PR1 protein; or, in other cases, the nucleic acid molecule encoding the chimeric antigen receptor may be located at a 3'-terminus of the nucleic acid molecule encoding the S1PR1 protein. of the modified immune effector cell

The nucleic acid molecule encoding the chimeric antigen receptor and the nucleic acid molecule encoding the S1PR1 protein may be linked by any feasible linker (for example, a cleavable peptide, such as IRES or 2A). For example, the nucleotide sequence of IRES may be as set forth in SEQ ID NO: 13 in the sequence list; and the nucleotide sequence of 2A may be as set forth in SEQ ID NO: 14 in the sequence list.

For example, the vector may sequentially include the nucleic acid molecule encoding the S1PR1 protein, the linker and the nucleic acid molecule encoding the chimeric antigen receptor from the 5'-terminus to the 3'-terminus. For example, the vector may sequentially include the nucleic acid molecule encoding the chimeric antigen receptor, the linker and the nucleic acid molecule encoding the S1PR1 protein from the 5' -terminus to the 3'-terminus. For example, the vector may include a nucleotide sequence as set forth in any one of SEQ ID NOs: 7 to 9.

The modified immune effector cell described in the present application includes the cases that different types of linkers and different linking sequences are included, all of which have the beneficial effects described in the present application. In the vector of the modified immune effector cell, when the nucleic acid molecule encoding the S1PR1 protein is attached to a N-terminus and a C-terminus of an antigen-encoding chimeric receptor, or when different types of linkers are administered, the modified immune effector cell have an anti-tumor effect in vivo or in vitro. For example, in an in-vitro killing experiment, the modified immune effector cell may kill a target cell, and has an increased capacity of secreting cytokines and an enhanced migration capacity. For example, in an in-vitro experiment, the modified immune effector cell may inhibit tumor growth, and has an enhanced effect of migrating from the lymph node.

The immune effector cell may include a T cell, B cell, a natural killer (NK) cell, a macrophage, a NKT cell, a monocyte, a dendritic cell, a granulocyte, a lymphocyte, a leukocyte, and/or a peripheral blood mononuclear cell. In some cases, the immune cell may include a T lymphocyte. The T lymphocyte may include a thymocyte, a natural T lymphocyte, an immature T lymphocyte, a mature T lymphocyte, a resting T lymphocyte, or an activated T lymphocyte. The T cell may be a helper T cell (Th), such as a helper T cell 1 (Th1) or a helper T cell 2 (Th2). The T lymphocyte may be CD4+ helper T cell (HTL; a CD4+ T cell), a cytotoxic T cell (CTL; a CD8+ T cell), a tumor infiltrating cytotoxic T cell (TIL; a CD8+ T cell), a CD4+/ CD8+ T cell, a CD4-/CD8-T cell, or any other T lymphocyte subtypes. The T lymphocyte may be a memory T cell, such as a central memory T cell (a T_{CM} cell), an effector memory T cell (a T_{EM} cell), a tissue resident memory T cell (T_{RM}), a virtual memory T cell (a T_{VM} cell), a stem memory T cell (a T_{SCM} cell), or other CD4+ or CD8+, which generally expresses CD45RO, but may also lack a CD45RA memory T cell.

In another aspect, the present application provides a cell population, which may contain the immune effector cell defined in the present application. In some cases, when the immune effector cell in the cell population reaches 20% or above (for example, 20% or above, 25% or above, 30% or above, 35% or above, 40% or above, 45% or above, 50% or above, 55% or above, 60% or above, 65% or above, 70% or above, 75% or above, 80% or above, 85% or above, 90% or above, 95% or above, or more), the cell population may be able to migrate towards a tumor site (for example, inside a tumor tissue). For example, in an in-vivo experiment, mice were injected with the cell population, and compared with the CAR T cell population that does not express S1PR1, an increase in the content of the T cells in the peripheral blood is detected in a cell population group described in the present application. For example, in an in-vitro experiment, the CAR T cell expressing the S1PR1 may migrate to a lower chamber. For another example, in an in-vivo experiment, mice were injected with the cell population to detect the content of the T cells in the peripheral blood. The T cells in a CAR T cell population expressing the S1PR1 tend to be distributed more inside the peripheral blood and tumor tissues; and the T cells in a CAR T cell population not expressing the S1PR1 tend to be distributed inside the peripheral blood, lymph nodes and tumor tissues.

### Pharmaceutical composition, method, uses and carrier

In another aspect, the present application provides a pharmaceutical composition. The pharmaceutical composition may contain the immune effector cell and/or the cell population according to the present application, and optionally a pharmaceutically acceptable carrier. In the present application, the term "pharmaceutically acceptable carrier" generally refers to any and all of solvents, dispersion media, coatings, antibacterial and antifungal agents, isotonic agents and absorption delay agents and others that are compatible with the administration of the immune cell and/or cell population according to the present application. Any conventional medium or reagent may be considered for use in the pharmaceutical composition of the present application, unless they are incompatible with the immune effector cell and/or the cell population described in the present application.

In some aspects, the method according to the present application may include administering one or more of the CAR and/or a vector containing the CAR to a host cell (for example, the immune effector cell). In some aspects, the method according to the present application may include administering one or more of the TCR and/or a vector containing the TCR to a host cell (for example, the immune effector cell). In some cases, the method according to the present application may include administering the S1PR1 and/or a vector containing the S1PR1 to a host cell (for example, the immune effector cell). The method according to the present application further includes delivering one or more vectors containing the CAR and/or TCR and the S1PR1 to the host cell (for example, the immune cell). In some aspects, the present application further provides a cell produced by the method and an organism (for example, an animal, a plant, or a fungus) including the cell or produced from the cells. In some cases, the CAR linked to S1PR1 may be delivered to the cell. Conventional viral and non-viral-based gene transfer methods may be used to introduce a nucleic acid sequence into a mammalian cell or a target tissue.

In another aspect, the present application provides a vector, which may contain the isolated nucleic acid molecule.

In the present application, the vector may be selected from one or more of a plasmid, a retroviral vector, and a lentiviral vector. For example, the vector according to the present application sequentially contains the following nucleotide sequences from the 5'-terminus: a gene encoding the S1PR1, a gene encoding the IRES, and a gene encoding the CAR. For another example, the vector according to the present application sequentially contains the following nucleotide sequences from the 5'-terminus: the gene encoding the S1PR1, a gene encoding the 2A, and the gene encoding the CAR. For another example, the vector according to the present application sequentially contains the following nucleotide sequences from the 5'-terminus: the gene encoding the CAR, a gene encoding the IRES, and a gene encoding the S1PR1. For another example, the vector according to the present application sequentially contains the following nucleotide sequences from the 5'-terminus: the gene encoding the CAR, the gene encoding the 2A, and the gene encoding the S1PR1. In addition, the vector may also contain other genes, for example, a marker gene that is allowed to select the vector in a suitable host cell and under a suitable condition. In addition, the vector may also contain an expression control element that allows a coding region to be expressed correctly in a suitable host. Such a control element is well known to those skilled in the art, which, for example, may include a promoter, a ribosome binding site, an enhancer and other control elements that regulate gene transcription or mRNA translation. In some embodiments, the expression control sequence is a regulatable element. A specific structure of the expression control sequence may vary depending on the function of the species or cell type, but generally includes a 5' non-transcribed sequence and 5' and 3' non-translated sequences, for example, a TATA box, a capped sequence, a CAAT sequence, etc., which participate in transcription and translation initiation, respectively. For example, the 5' non-transcribed expression control sequence may include a promoter region, and the promoter region may include a promoter sequence for functionally linked to the nucleic acid for transcriptional control. The one or more nucleic acid molecules described in the present application may be operably linked to the expression control element.

Methods for non-viral delivery of the nucleic acid include lipofection, nuclear transfection, microinjection, gene gun, viral particles, liposomes, immunoliposomes, polycations or lipid nucleic acid conjugates, naked DNA, artificial virions, and reagents for enhancing DNA uptake. The nucleic acid may be delivered by using an RNA or DNA virus-based system. For example, by virtue of the property of the virus capable of targeting a specific cell in vivo, the virus is effectively payloaded and transported to a nucleus. The viral vector may be administered directly to a patient (in vivo) or in an indirect form. For example, the virus is used to treat a cell in vitro, and then the treated cell is administered to the patient (in vitro). A conventional virus-based system may include a retroviral vector, a lentiviral vector, an adenovirus vector, an adeno-associated virus vector, and a herpes simplex virus vector, for gene transfer. In some cases, the retrovirus, the lentivirus, and the adeno-associated virus may be used to transfer and integrate a gene into a host genome, allowing the inserted gene to be expressed for a long time. The lentiviral vector is a retroviral vector that may transduce or infect a non-dividing cell and typically produce a higher viral titer. The lentiviral vector may contain a long terminal repeat 5'LTR and a truncated 3'LTR, RRE, a rev response element (cPPT), a central termination sequence (CTS), and/or a post-translational regulatory element (WPRE). The molecule may be constructed on the lentiviral vector by BamHI and XbaI digestion. The choice of a retroviral gene transfer system will depend on the target tissue.

The method according to the present application may include introducing the vector described in the present application into the immune effector cell. For example, the vector described in the present application may be introduced in the immune effector cell such as a T cell, a lymphocyte, a granulocyte and/or a peripheral blood mononuclear cell. In some embodiments, each type of or each cell may contain one or one type of the vector described in the present application. In some embodiments, each type of or each cell may contain a plurality of (for example, 2 or more) or a plurality of types of (for example, 2 or more) vectors described in the present application. For example, the vector described in the present application may be introduced into the cell. For example, the immune effector cell may be transfected by the retroviral vector to integrate a viral genome with the nucleic acid encoding the fusion protein into a host genome, thereby ensuring the long-term and stable expression of the target gene. In the present application, the vector with the nucleic acid encoding the fusion protein described in the present application may be introduced into the cell by methods known in the art, such as electroporation (a Neon electroporator) and liposome transfection.

In another aspect, the present application provides uses of the immune effector cell as defined, the cell population as defined and/or the pharmaceutical composition as defined in the preparation of drugs for the treatment of tumors. In some cases, the tumor may include a solid tumor. For example, the tumors comprise pancreatic cancer, glioma, liver cancer and/or colon cancer.

Not wishing to be bound by any particular theory, the following examples are merely to illustrate the fusion protein, preparation methods and uses etc. according to the present application, and are not intended to limit the scope of the present invention.

### Examples

### Example 1 Construction and packaging of lentiviral vector

### 1.1 Construction of lentiviral vector

S1PR1 (with the Accession No. of NM_001400 in NCBI GenBank), IRES (with a nucleotide sequence as set forth in SEQ ID NO: 13 in the sequence list), and a GFP gene (with a nucleotide sequence as set forth in SEQ ID NO: 15 in the sequence list) were connected in sequence through synthesis using a gene-wide synthesis method; a BamHI restriction site sequence was added downstream of S1PR1; an XbaI restriction site sequence was added upstream of the GFP gene, and then cloned into a place between two restriction site of a vector pLVX-EF1α-IRES-Puro EcoRI/MluI; and then, a S1PR1-IRES-GFP (with a nucleotide sequence as set forth in SEQ ID NO: 10) plasmid was constructed.

An anti-GPC3 CAR gene GC33CAR was prepared through gene-wide synthesis, which was an anti-GPC3 single-chain antibody GC33 (with an amino acid sequence as set forth in SEQ ID NO: 1), and a hinge region CD8a, a transmembrane region CD8, an intracellular region CD137 (4-1BB), and an intracellular region CD3z were serially connected in sequence. A nucleotide sequence of GC33CAR was as set forth in SEQ ID NO: 3. GC33CAR was used to replace S1PR1 and GFP in S1PR1-IRES-GFP respectively to construct GC33CAR-IRES-GFP (with a nucleotide sequence as set forth in SEQ ID NO: 11) and S1PR1-IRES-GC33CAR (with a nucleotide sequence as set forth in SEQ ID NO: 12) plasmids.

2A (with a nucleotide sequence as set forth in SEQ ID NO: 14) was used to replace IRES in S1PR1-IRES-GC33CAR, and a vector was inserted to prepare a S1PR1-2A-GC33CAR (with a nucleotide sequence as set forth in SEQ ID NO: 8) plasmid.

S1PR1 was used to replace GFP in GC33CAR-IRES-GFP, and a vector was inserted to prepare a GC33CAR-IRES-S1PR1 (with a nucleotide sequence as set forth in SEQ ID NO: 7) plasmid.

The GC33CAR gene was inserted into the vector to prepare a GC33CAR plasmid.

An anti-MSLN CAR gene P4G2CAR was prepared through gene-wide synthesis, which was an anti-MSLN single-chain antibody P4G2 (with an amino acid sequence as set forth in SEQ ID NO: 2), and a hinge region CD8a, a transmembrane region CD8, an intracellular region CD137 (4-1BB), and an intracellular region CD3z were serially connected in sequence. A nucleotide sequence of P4G2CAR was as shown in SEQ ID NO: 4. P4G2CAR was used to replace GC3CAR in S1PR1-2A-GC33CAR to prepare a S1PR1-2A-P4G2CAR (with a nucleotide sequence as set forth in SEQ ID NO: 9) plasmid.

The P4G2 gene was inserted into the vector to prepare a P4G2 plasmid.

### 1.2 Packaging of lentivirus

A lentivirus was packaged through a three-plasmid system.

### Example 2 Verification of CAR expression

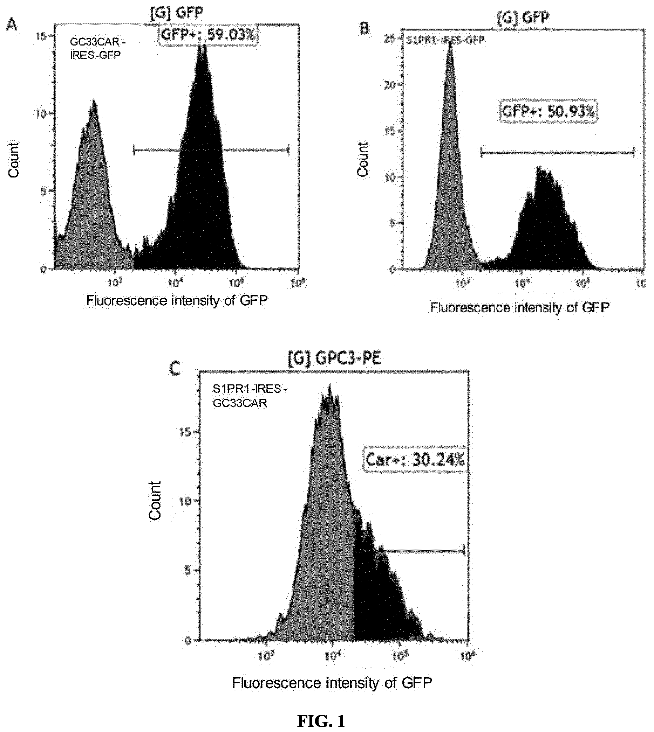

CAR-T cells were constructed by using GC33CAR-IRES-GFP, S1PR1-IRES-GC33CAR, and S1PR1-IRES-GFP lentiviruses to infect and activate PBMC cells (activated by CD3 and CD28). Flow cytometry was used to detect a CAR-positive rate of the virus-infected CAR-T cells, where the CAR-positive rate of the immune T cells transfected with S1PR1-IRES-GFP was alternatively detected by GFP. The results were as shown in Table 1 and FIG.1. The results showed that the CAR-positive rate of the CAR-T cells containing GC33CAR-IRES-GFP was 56.68% (FIG. 1A); the positive rate of the immune T cells transfected with S1PR1-IRES-GFP (as a control) was 50.93% (FIG. 1B); and the CAR-positive rate of the CAR-T cells containing S1PR1-IRES- GC33CAR was 30.24% (FIG. 1C), which was the lowest.

**Table 1 Positive rates of CAR-T (or T) cells**

| | [GFP+] % Gated | [CAR+] % Gated |
|---|---|---|
| GC33CAR-IRES-GFP | 59.03 | 56.68 |
| S1PR1-IRES-GFP | 50.93 | 0.2 |
| S1PR1-IRES-GC33CAR | 0.69 | 30.24 |

### Example 3 Verification of S1PR1 migration function in vitro

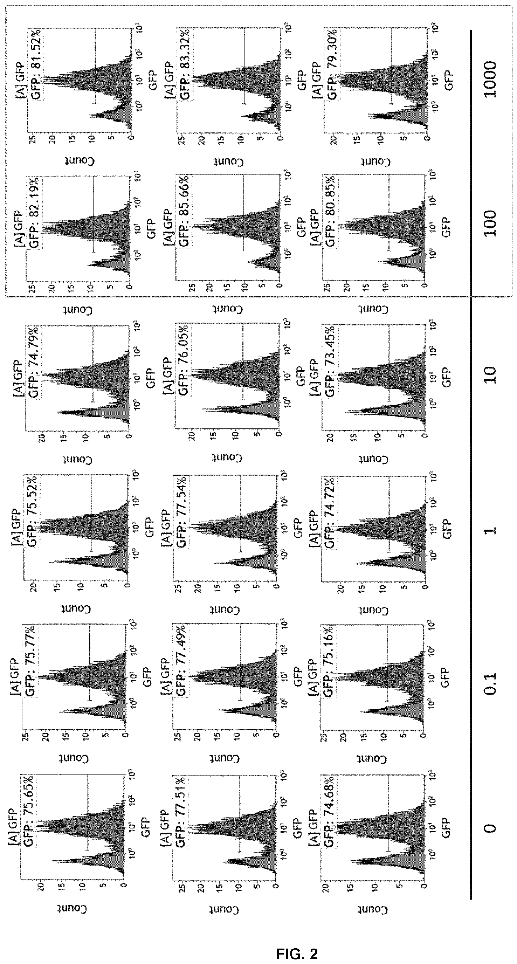

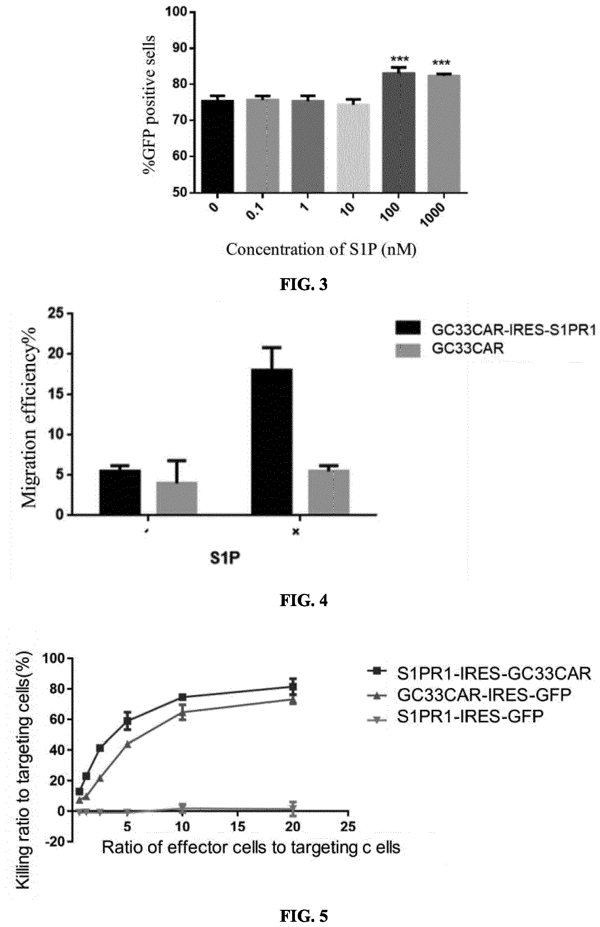

The migration capacity of the CAR-T cells was verified under gradient concentrations of SIP (0, 1, 10, 100, 1000 nM) by a Transwell experiment. 200 µl of S1PR1-IRES-GFP-transfected immune T cells (2.5×10⁶ cells) with a GFP-positive rate of 70% was added into an upper chamber of a 3415 Transwell chamber, with 1640 as a culture solution; 500 µl of SIP at gradient concentrations was added to a lower chamber, with 1.5% FBS +1640 as a culture solution. The immune T cells were incubated in an incubator for 3 hours, and the GFP-positive rate of the cells that had migrated to the lower chamber was detected by flow cytometry. The results showed that with the SIP treatment at 100 nM and 1000 nM, the proportion of positive CAR-T cells that had migrated to the lower chamber was significantly increased. That is, the immune T cells expressing the S1PR1 could enhance the migration capacity of the cells affected by SIP (FIG. 2 and FIG. 3).

1×10⁶ immune T cells transfected with GC33CAR or GC33CAR-IRES-S1PR1 were put in the upper chamber of the 3415 Transwell chamber, with 1640 as the culture solution; 500µl of 100nM SIP was added to the lower chamber, with 1640 as the culture solution; and incubation was performed in the incubator for 3 hours. No SIP was added as a control. The amount of cells in the lower chamber was calculated with a cell counter, with the migration capacity% = the amount of cells in the lower chamber/(1×10⁶)×100%. The results showed that the CAR T cells expressing the S1PR1 could enhance the cell migration capacity, and the transfer efficiency was high when SIP is positive (FIG. 4).

### Example 4 Verification of killing capacity against target cells

### 4.1 Killing capacity of T cells infected with S1PR1-IRES-GFP, GC33CAR-IRES-GFP and S1PR1-IRES-GC33CAR

The killing capacity of the T (or CAR-T) cells infected with the S1PR1-IRES-GFP, GC33CAR-IRES-GFP and S1PR1-IRES-GC33CAR lentiviruses against target cells Huh7 (purchased from the Cell Bank of Chinese Academy of Sciences) expressing a luciferase protein (Luc) was verified by the T cell killing experiment. The ratio of effector cells to target cells included: 20:1, 10:1, 5:1, 2.5:1, 1.25:1 and 0.625:1. The following groups were set up: a blank control group of DMEM (2% FBS) culture solution; a CAR-T effector cell group; an effector cell and Huh7 target cell group, where the target cells were 1×10⁴ cells per well, and the effector cells were added based on the effector cell/target cell ratio; a target cell group (Huh7) and a maximum lysis rate group (Huh7-max), with 1×10⁴ cells per well. Incubation was performed at 37°C for 4 hours. Treatment was performed for 3.5 hours, and 20 µl of lysis buffer was added to each well for the Huh7-max group. The incubation was continued for half an hour, and the plate was centrifuged at 300 g for 5 minutes. Based on the original plate order, 50 µl of supernatant was extracted from each well and put into a COSTAR 3300 well plate. 50 µl of substrate was added to allow reaction for 30 minutes at normal temperature, and then, 50 µl of STOP Buffer (cytoTox 96 Non-radioactive Cytotoxicity kit, promega) was added. An OD490 reading was detected on the computer, and the killing efficiency of the CAR-T cells against the target cells was calculated with a formula as follows: specific killing% = (experimental group - self-release of effector cells - self-release of target cells)/(maximum release of target cells - self-release of target cells) × 100%. The results showed that the CAR-T cells containing (or transfected with) S1PR1-IRES-GC33CAR had the strongest killing capacity against the target cells, followed by the CAR-T cells containing GC33CAR-IRES-GFP, while the CAR-T cells transfected with S1PR1-IRES-GFP had no significant killing capacity against the target cells (FIG. 5).

### 4.2 Killing capacity of T cells infected with GC33CAR-IRES-S1PR1, S1PR1-2A-GC33CAR, and GC33CAR

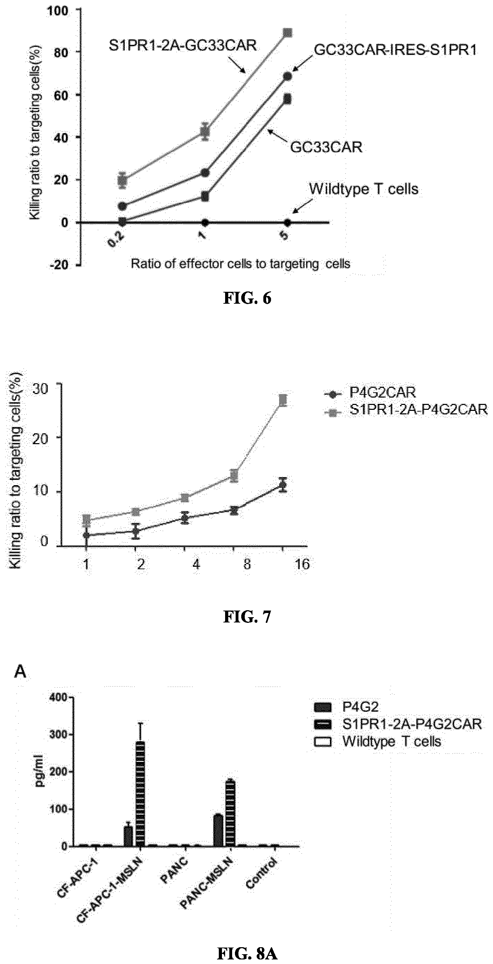

Based on the method in 4.1, the killing capacity of the T (or CAR-T) cells infected with GC33CAR-IRES-S1PR1, S1PR1-2A-GC33CAR and GC33CAR lentiviruses against the target cells Huh7-Luc was verified. GC33CAR-infected T cells and wild-type T cells were taken as controls. The results were shown in FIG. 6. The CAR cells expressing S1PR1 at the 5'-terminus and the CAR T cells expressing S1PR1 at the 3'-terminus both exhibited the killing capacity against the target cells, and the T cells with different linking peptides between S1PR1 and CAR showed the killing capacity against the target cells.

### 4.3 Killing capacity of T cells infected with S1PR1-2A-P4G2CAR

Based on the method in 4.1, the killing capacity of the T cells infected with S1PR1-2A-P4G2CAR lentiviruses against the target cells Huh7-Luc was verified. The T cells infected with P4G2CAR were taken as a control. The results were shown in FIG. 7. The CAR T cells expressing S1PR1 exhibited the killing capacity against the target cells, which is higher than that of the control T cells.

### Example 5 In-vitro verification of cytokine secretion

The CAR-T cells were constructed by using P4G2 and S1PR1-2A-P4G2CAR lentivirus to infect and activate PBMC cells (activated by CD3 and CD28) so as to verify the capacity of the T cells to secrete cytokines in vitro.

In short, MSLN-expressing CF-APC-1 cells CF-APC-1-MSLN were first constructed; the nucleotide sequence of MSLN was acquired from the NCBI database under the Accession No. NM_005823; MSLN-IRES-puro was synthesized; a lentivirus overexpressing MSLN- IRES-puro was packaged; CF-APC-1 cells (from the Cell Bank of Chinese Academy of Sciences, TCHu112) were infected with the prepared lentivirus; and after 48 hours, screening was performed with 5ug/ml puromycin to obtain a stable transgenic strain.

CF-APC-1 cells (from the Cell Bank of Chinese Academy of Sciences, TCHu112), CF-APC-1-MSLN cells, human pancreatic cancer cells (PANC) (from the Cell Bank of Chinese Academy of Sciences), and PANC cell expressing MSLN (from the Cell Bank of Chinese Academy of Sciences, TCHu 98) as target cells were washed, suspended in a DMEM (containing 2% FBS) medium at 1×10⁵ cells/mL, and the respective target cells were added to a 96-well plate at 1×10⁵ cells/well. The CAR-T cells were washed and suspended in the DMEM (containing 2% FBS) medium at 1×10⁵ cells/well. In addition, wild-type T cells that do not express CAR were taken as a control. The 96-well plate was incubated at 37°C for 24 hours. Then, the supernatant was harvested, and a human TH1/TH2 cytokine detection kit (BD CBA, Cat#551809) was used for the flow cytometric determination of IL-2 and IFN-γ.

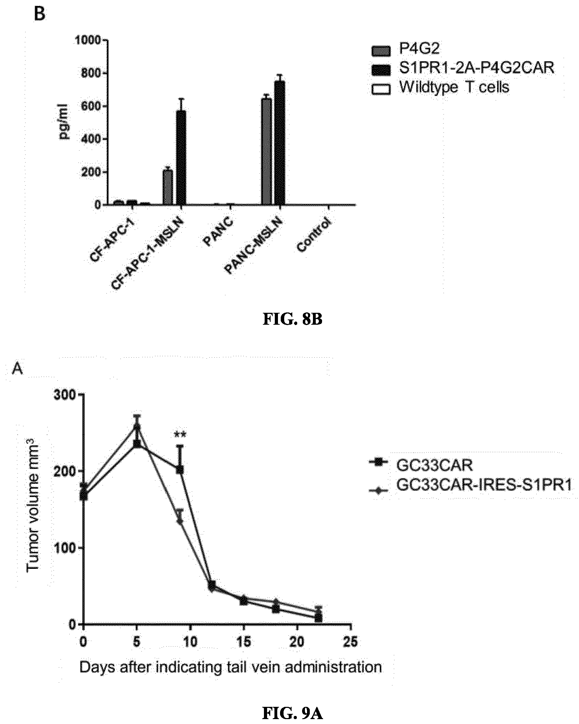

The results showed that under the stimulation of the target cells expressing MSLN, the T cells expressing S1PR1-2A-P4G2CAR exhibited an enhanced capacity of secreting the cytokines IL-2 (FIG. 8A) and IFN-γ (FIG. 8B) compared with the T cell expressing only P4G2CAR.

### Example 6 Experimental verification of tumor inhibition in vivo

The CAR-T cells were constructed by using GC33CAR and GC33CAR-IRES- S1PR1 lentiviruses to infect and activate PBMC cells (activated by CD3 and CD28).

In short, self-constructed target tumor cells expressing luciferase were inoculated into NSG mice tumors induced by subcutaneous injection; and 2×10⁶ above-mentioned target cells were resuspended in 100 µl of phosphate buffered saline solution and injected subcutaneously. Or, the self-constructed target tumor cells expressing luciferase were inoculated into NSG mice tumors induced by intravenous injection; and 2×10⁶ above-mentioned target cells were resuspended in 100 µl of phosphate buffered saline solution and injected to the mice subcutaneously. The tumors were measured by a vernier caliper every 3-4 days, with the tumor volume = length^{∗}width^{∗}width/2. After 14 days of tumor formation, the CAR T cells washed with PBS were resuspended in a serum-free culture solution, to adjust the cell density to 1×10⁷/ml for intravenous injection (i.v.) into the mice at 200 µl/mouse, or to adjust the cell density to 1×10⁸/ml for intratumoral injection (i.t.) into the mice at 50 µl/mouse. Wild-type T cells were taken as a control. After the injection, the tumor volume was measured every 3 days. The tumor volume was calculated as follows: tumor volume (mm³) = 0.5 ×long tumor diameter × short tumor diameter². On Day 28, the peripheral blood of the mice was collected and measured for the amount of CD3⁺ cells by flow cytometry.

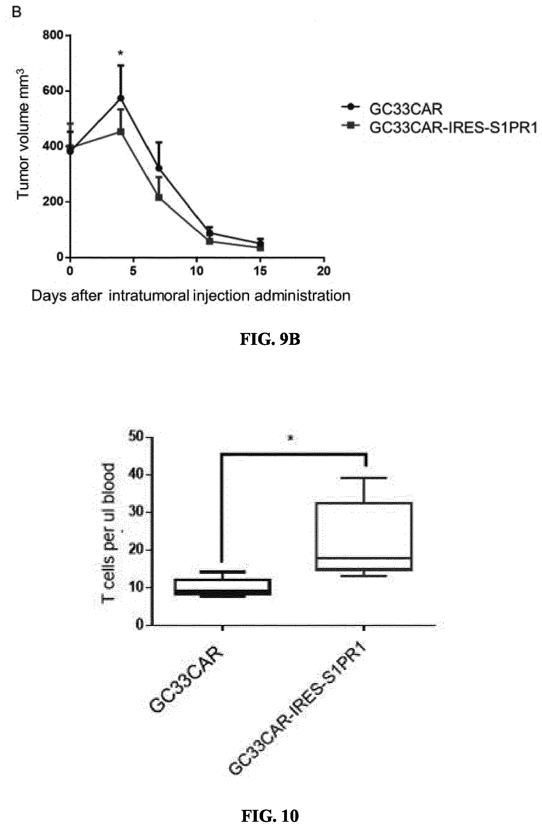

The results showed that both the GC33CAR-transfected T cells and the GC33CAR-IRES-S1PR1-transfected T cells may effectively inhibit the tumor growth (FIG. 8). FIGs. 9A-9B show that the tumor volumes of mice decreased since the CAR T cells were injected by different injection means.

### Example 7 Verification of S1PR1 migration function in vivo

Mice models were established based on the method of Example 6, grouped, and injected with GC33CAR T cell and GC33CAR-IRES-S1PR1 T cell after 14 days since tumor formation. On Day 28, the peripheral blood of the mice was collected and measured for the amount of CD3+ cells by flow cytometry.

The results showed that the amount of T cells in the peripheral blood of the mice administered with GC33CAR-IRES-S1PR1-transfected T cell was greater than that in the group administered with the GC33CAR-transfected T cells (FIG. 10). The CAR T cells expressing S1PR1 can enhance the cell migration capacity.

### Example 8 Verification of synergistic effect between S1PR1 and CAR

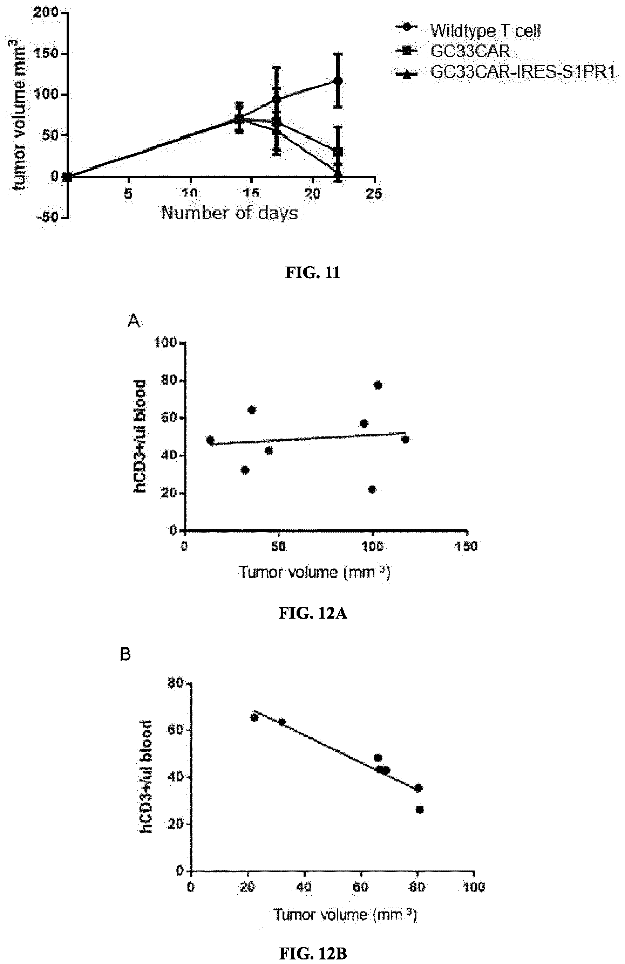

Mice tumor models were established based on the methods of Example 7 and 8, and injected with the CAR T cells after 14 days since tumor formation. Then, the tumor volume was measured every 3 days, and the peripheral blood was collected and measured for the amount of CD3⁺ T cells. FIG. 11 shows the change in tumor volume since the tumor formation in the mice. After the CAR T cells according to the present application were injected, the tumor volume decreased. In the GC33CAR-IRES-S1PR1-transfected T cell group, the amount of CD3⁺ T cells in the peripheral blood was negatively correlated with the tumor volume (FIG. 12B), which might indicate that the GC33CAR-IRES-S1PR1-transfected T cells tended to be distributed more in the peripheral blood and tumor tissues. There is no such correlation with respect to the GC33CAR-transfected T cells (FIG. 12A), which might be due to the fact that the GC33CAR-transfected T cells tended to be distributed in the peripheral blood, lymph nodes and tumor tissues. These data indicate that S1PR1 and CAR show an anti-tumor synergistic effect in vivo.

## Claims

1. A modified immune effector cell, in which the expression and/or activity of a S1PR1 protein is up-regulated compared with an unmodified immune effector cell.

2. The immune effector cell of claim 1, wherein the immune effector cell comprises a T cell, a lymphocyte, a granulocyte and/or a peripheral blood mononuclear cell.

3. The immune effector cell of any one of claims 1 to 2, wherein the immune effector cell comprises a memory T cell.

4. The immune effector cell of any one of claims 1 to 3, wherein in said modified immune cell, the expression and/or activity of a nucleic acid molecule encoding the S1PR1 protein is up-regulated.

5. The immune effector cell of any one of claims 1 to 4, wherein said modified immune cell additionally contains the S1PR1 protein.

6. The immune effector cell of any one of claims 1 to 5, wherein said modified immune effector cell additionally contains the nucleic acid molecule encoding said S1PR1 protein.

7. The immune effector cell of any one of claims 1 to 6, wherein said modified immune effector cell contains a vector, which comprises the nucleic acid molecule encoding the S1PR1 protein.

8. The immune effector cell of any one of claims 1 to 7, wherein said immune effector cell expresses a chimeric antigen receptor CAR and/or a T cell receptor (TCR).

9. The immune effector cell of any one of claims 1 to 8, wherein said modified immune effector cell contains a vector, which comprises said nucleic acid molecule encoding the chimeric antigen receptor.

10. The immune effector cell of claim 9, wherein said nucleic acid molecule encoding the chimeric antigen receptor and said nucleic acid molecule encoding the S1PR1 protein are located in the same vector.

11. The immune effector cell of claim 10, wherein said nucleic acid molecule encoding the chimeric antigen receptor is located at a 5'-terminus of said nucleic acid molecule encoding the S1PR1 protein; or, said nucleic acid molecule encoding the chimeric antigen receptor is located at a 3'-terminus of said nucleic acid molecule encoding the S1PR1 protein.

12. The immune effector cell of any one of claims 8 to 11, wherein the chimeric antigen receptor comprises a chimeric antigen receptor targeting a tumor-specific antigen, wherein the tumor-specific antigen is selected from the group consisting of: EpCAM, Mesothelin (MSLN), CEA, IL13, PDPN, VEGF, EGFR, EGFRvIII, PSMA, FAP, CD171, GD2, Glypican-2, Glypican-3, HER2, HPV antigen, cyclin D1, p53, MMP-7, IL13Ralpha2, MMP-2, MUC-1, G250, L1CAM, ROR1, and GPC3.

13. The immune effector cell of any one of claims 8 to 12, wherein said chimeric antigen receptor includes an antigen-binding domain that specifically binds to said tumor-specific antigen.

14. The immune effector cell of claim 13, wherein said antigen-binding domain comprises a single-chain antibody.

15. The immune effector cell of any one of claims 13 to 14, wherein the antigen-binding domain comprises a single-chain antibody targeting GPC3 or MSLN.

16. The immune effector cell of claim 15, wherein the single-chain antibody includes an amino acid sequence as set forth in any one of SEQ ID NOs: 1 to 2.

17. The immune effector cell of any one of claims 8 to 16, wherein said chimeric antigen receptor includes a transmembrane domain.

18. The immune effector cell of claim 17, wherein said transmembrane domain includes a transmembrane domain derived from a protein selected from the group consisting of: CD3ζ, CD28, 4-1BB, OX40, SLAMF4, CD127, NKG2D, ICOS, and FcγRIIIa.

19. The immune effector cell of any one of claims 8 to 18, wherein said chimeric antigen receptor includes a costimulatory domain.

20. The immune effector cell of claim 19, wherein said costimulatory domain includes a costimulatory domain selected from the following proteins or a combination thereof: CD137, CD28, OX40, ICOS, DAP10, 2B4, CD27, CD30, CD40, CD40L, TIM1, CD226, DR3, SLAM, NKG2D, CD244, FceRIy, BTLA, GITR, HVEM, CD2, NKG2C, LIGHT, and DAP12.

21. The immune effector cell of any one of claims 8 to 20, wherein said chimeric antigen receptor includes a hinge region, which connects said antigen-binding domain and said transmembrane domain.

22. The immune effector cell of claim 21, wherein said hinge region includes a hinge region derived from a protein selected from the group consisting of: CD8, CD28, IgG, 4-1BB, CD4, CD27, CD7, PD-1, and CH2CH3.

23. The immune effector cell of any one of claims 8 to 22, wherein said chimeric antigen receptor includes an amino acid sequence as set forth in any one of SEQ ID NOs: 5 to 6.

24. The immune effector cell of any one of claims 8 to 23, comprising a nucleic acid molecule encoding said chimeric antigen receptor.

25. The immune effector cell of claim 24, wherein a vector including said nucleic acid molecule encoding the chimeric antigen receptor and said nucleic acid molecule encoding the S1PR1 protein contains a nucleotide sequence as set forth in any one of SEQ ID NOs: 7 to 9.

26. A cell population, containing the immune effector cell of any one of claims 1 to 25.

27. A pharmaceutical composition containing the immune effector cell of any one of claims 1 to 25 and/or the cell population of claim 26, and optionally a pharmaceutically acceptable carrier.

28. Use of the immune effector cell of any one of claims 1 to 25, the cell population of claim 26 and/or the pharmaceutical composition of claim 27 in the preparation of a drug for the treatment of a tumor.

29. The use of claim 28, wherein said tumor comprises a solid tumor.

30. The use of any one of claims 28 to 29, wherein said tumor comprises pancreatic cancer, glioma, liver cancer and/or colon cancer.


Sequence Listing

<110>  SHANGHAI PHARMACEUTICALS HOLDING CO., LTD

<120>  IMMUNE CELL CONTAINING TUMOR ANTIGEN RECOGNITION RECEPTOR AND
APPLICATION THEREOF

<130>  0124-PA-002EP

<160>  15

<170>  PatentIn version 3.5

<210>  1
<211>  242
<212>  PRT
<213>  Artificial Sequence

<220>
<223>  GC33

<400>  1

Asp Val Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Pro Thr Phe Gly Gln Gly Thr Lys Leu Glu Ile Lys
            100                 105                 110

Gly Gly Gly Gly Ser Gly Gly Gly Gly Ser Gly Gly Gly Gly Ser Gln
        115                 120                 125

Val Gln Leu Val Gln Ser Gly Ala Glu Val Lys Lys Pro Gly Ala Ser
    130                 135                 140

Val Lys Val Ser Cys Lys Ala Ser Gly Tyr Thr Phe Thr Asp Tyr Glu
145                 150                 155                 160

Met His Trp Val Arg Gln Ala Pro Gly Gln Gly Leu Glu Trp Met Gly
                165                 170                 175

Ala Leu Asp Pro Lys Thr Gly Asp Thr Ala Tyr Ser Gln Lys Phe Lys
            180                 185                 190

Gly Arg Val Thr Leu Thr Ala Asp Glu Ser Thr Ser Thr Ala Tyr Met
        195                 200                 205

Glu Leu Ser Ser Leu Arg Ser Glu Asp Thr Ala Val Tyr Tyr Cys Thr
    210                 215                 220

Arg Phe Tyr Ser Tyr Thr Tyr Trp Gly Gln Gly Thr Leu Val Thr Val
225                 230                 235                 240

Ser Ser

<210>  2
<211>  258
<212>  PRT
<213>  Artificial Sequence

<220>
<223>  P4G2

<400>  2

Gln Val Gln Leu Gln Gln Ser Gly Pro Gly Leu Val Thr Pro Ser Gln
1               5                   10                  15

Thr Leu Ser Leu Thr Cys Ala Ile Ser Gly Asp Ser Val Ser Ser Asn
            20                  25                  30

Ser Ala Thr Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Met Ser Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Gly Met Met Thr Tyr Tyr Tyr Gly Met Asp Val
            100                 105                 110

Trp Gly Gln Gly Thr Thr Val Thr Val Ser Ser Gly Ile Leu Gly Ser

115                 120                 125

Gly Gly Gly Gly Ser Gly Gly Gly Gly Ser Gly Gly Gly Gly Ser Gln
    130                 135                 140

Pro Val Leu Thr Gln Ser Ser Ser Leu Ser Ala Ser Pro Gly Ala Ser
145                 150                 155                 160

Ala Ser Leu Thr Cys Thr Leu Arg Ser Gly Ile Asn Val Gly Pro Tyr
                165                 170                 175

Arg Ile Tyr Trp Tyr Gln Gln Lys Pro Gly Ser Pro Pro Gln Tyr Leu
            180                 185                 190

Leu Asn Tyr Lys Ser Asp Ser Asp Lys Gln Gln Gly Ser Gly Val Pro
        195                 200                 205

Ser Arg Phe Ser Gly Ser Lys Asp Ala Ser Ala Asn Ala Gly Val Leu
    210                 215                 220

Leu Ile Ser Gly Leu Arg Ser Glu Asp Glu Ala Asp Tyr Tyr Cys Met
225                 230                 235                 240

Ile Trp His Ser Ser Ala Ala Val Phe Gly Gly Gly Thr Gln Leu Thr
                245                 250                 255

Val Leu

<210>  3
<211>  1518
<212>  DNA
<213>  Artificial Sequence

<220>
<223>  GC33CAR

<400>  3
atggccttac cagtgaccgc cttgctcctg ccgctggcct tgctgctcca cgccgccagg      60

ccggacgtgg tgatgaccca gagcccttta tctttacccg ttacacccgg tgagcccgct     120

agcatctctt gtagaagcag ccagtcttta gtgcacagca acggcaacac atatttacac     180

tggtatttac agaagcccgg tcagagcccc cagctgctga tctacaaggt gagcaatcgt     240

ttctccggcg tgcccgatag attcagcggc agcggctccg gaaccgactt cactttaaag     300

atcagcagag tggaggccga ggacgtgggt gtgtactact gctcccagaa cacccacgtg     360

ccccctacat tcggtcaagg taccaaactg gagatcaaag gtggcggtgg ctcgggcggt     420

ggtgggtcgg gtggcggcgg atctcaagtt cagctggtgc agagcggcgc cgaggtcaaa     480

aaacccggag ccagcgtgaa ggtgtcttgt aaagccagcg gctacacctt taccgactat 540

gagatgcact gggtgagaca agctcccggt caaggtctgg aatggatggg cgctttagac 600

cccaagactg gtgacacagc ctactcccag aagttcaagg gtcgtgtgac tttaacagcc 660

gacgagtcca ccagcacagc ctacatggaa ctgagctctt taaggagcga ggacaccgcc 720

gtgtattact gcactcgttt ctacagctac acctactggg gccaaggtac tttagtgaca 780

gtgagcagct tcgtgccggt cttcctgcca gcgaagccca ccacgacgcc agcgccgcga 840

ccaccaacac cggcgcccac catcgcgtcg cagcccctgt ccctgcgccc agaggcgtgc 900

cggccagcgg cggggggcgc agtgcacacg agggggctgg acttcgcctg tgatatctac 960

atctgggcgc ccttggccgg gacttgtggg gtccttctcc tgtcactggt tatcaccctt 1020

tactgcaacc acaggaaccg tttctctgtt gttaaacggg gcagaaagaa gctcctgtat 1080

atattcaaac aaccatttat gagaccagta caaactactc aagaggaaga tggctgtagc 1140

tgccgatttc cagaagaaga agaaggagga tgtgaactga gagtgaagtt cagcaggagc 1200

gcagacgccc ccgcgtacca gcagggccag aaccagctct ataacgagct caatctagga 1260

cgaagagagg agtacgatgt tttggacaaa agaagaggcc gggaccctga gatgggggga 1320

aagccgagaa ggaagaaccc tcaggaaggc ctgtacaatg aactgcagaa agataagatg 1380

gcggaggcct acagtgagat tgggatgaaa ggcgagcgcc ggaggggcaa ggggcacgat 1440

ggcctttacc agggtctcag tacagccacc aaggacacct acgacgccct tcacatgcag 1500

gccctgcccc ctcgctaa 1518

<210> 4
<211> 882
<212> DNA
<213> Artificial Sequence

<220>
<223> P4G2CAR

<400> 4
atggctctgc ccgtgaccgc tctgctgctg cctctcgctc tgctgctgca tgccgccaga 60

cccggctccc aagtgcaact ccagcaatcc ggacccggac tggtgacacc ctcccaaaca 120

ctgtccctca catgcgccat cagcggagat tccgtgtcct ccaacagcgc tacatggaat 180

tggattagac agagcccttc cagaggactg gagtggctcg gaagaacata ctacagatcc 240

aagtggtata acgactacgc cgtgtccgtg aagtctagaa tgagcatcaa ccccgacacc 300

agcaagaacc agttttctct gcagctgaac agcgtgacac ccgaggacac cgctgtgtac 360

tactgcgcca gaggcatgat gacctactac tacggcatgg acgtctgggg acaaggcacc 420

accgtgacag tgagcagcgg cattctgggc tccggaggag gcggaagcgg aggcggaggc 480

agcggaggcg gcggaagcca acccgtgctc acacaaagct cctctctgag cgctagcccc 540

ggcgcctccg cttctctgac atgtacactg aggagcggca tcaacgtcgg accctataga 600

atctactggt accagcagaa gcccggcagc cccccccaat atctgctgaa ctacaagtcc 660

gactccgaca agcagcaagg cagcggcgtg ccttctagat tcagcggcag caaggacgct 720

agcgccaacg ccggagtgct gctgatctcc ggactgagaa gcgaggacga ggccgactac 780

tattgcatga tctggcactc cagcgccgct gtctttggag gaggcacaca gctgacagtg 840

ctgagcgcca gcttcgtgcc cgtgttcctc cccgccaaac cc 882

<210> 5
<211> 505
<212> PRT
<213> Artificial Sequence

<220>
<223> GC33CAR

<400> 5

Met Ala Leu Pro Val Thr Ala Leu Leu Leu Pro Leu Ala Leu Leu Leu
1               5                   10                  15

His Ala Ala Arg Pro Asp Val Val Met Thr Gln Ser Pro Leu Ser Leu
            20                  25                  30

Pro Val Thr Pro Gly Glu Pro Ala Ser Ile Ser Cys Arg Ser Ser Gln
        35                  40                  45

Ser Leu Val His Ser Asn Gly Asn Thr Tyr Leu His Trp Tyr Leu Gln
    50                  55                  60

Lys Pro Gly Gln Ser Pro Gln Leu Leu Ile Tyr Lys Val Ser Asn Arg
65                  70                  75                  80

Phe Ser Gly Val Pro Asp Arg Phe Ser Gly Ser Gly Ser Gly Thr Asp
                85                  90                  95

Phe Thr Leu Lys Ile Ser Arg Val Glu Ala Glu Asp Val Gly Val Tyr
            100                 105                 110

Tyr Cys Ser Gln Asn Thr His Val Pro Pro Thr Phe Gly Gln Gly Thr
        115                 120                 125

Lys Leu Glu Ile Lys Gly Gly Gly Gly Ser Gly Gly Gly Gly Ser Gly
    130                 135                 140

Gly Gly Gly Ser Gln Val Gln Leu Val Gln Ser Gly Ala Glu Val Lys

145                 150                 155                 160

Lys Pro Gly Ala Ser Val Lys Val Ser Cys Lys Ala Ser Gly Tyr Thr
                165                 170                 175

Phe Thr Asp Tyr Glu Met His Trp Val Arg Gln Ala Pro Gly Gln Gly
            180                 185                 190

Leu Glu Trp Met Gly Ala Leu Asp Pro Lys Thr Gly Asp Thr Ala Tyr
        195                 200                 205

Ser Gln Lys Phe Lys Gly Arg Val Thr Leu Thr Ala Asp Glu Ser Thr
    210                 215                 220

Ser Thr Ala Tyr Met Glu Leu Ser Ser Leu Arg Ser Glu Asp Thr Ala
225                 230                 235                 240

Val Tyr Tyr Cys Thr Arg Phe Tyr Ser Tyr Thr Tyr Trp Gly Gln Gly
                245                 250                 255

Thr Leu Val Thr Val Ser Ser Phe Val Pro Val Phe Leu Pro Ala Lys
            260                 265                 270

Pro Thr Thr Thr Pro Ala Pro Arg Pro Pro Thr Pro Ala Pro Thr Ile
        275                 280                 285

Ala Ser Gln Pro Leu Ser Leu Arg Pro Glu Ala Cys Arg Pro Ala Ala
    290                 295                 300

Gly Gly Ala Val His Thr Arg Gly Leu Asp Phe Ala Cys Asp Ile Tyr
305                 310                 315                 320

Ile Trp Ala Pro Leu Ala Gly Thr Cys Gly Val Leu Leu Leu Ser Leu
                325                 330                 335

Val Ile Thr Leu Tyr Cys Asn His Arg Asn Arg Phe Ser Val Val Lys
            340                 345                 350

Arg Gly Arg Lys Lys Leu Leu Tyr Ile Phe Lys Gln Pro Phe Met Arg
        355                 360                 365

Pro Val Gln Thr Thr Gln Glu Glu Asp Gly Cys Ser Cys Arg Phe Pro
    370                 375                 380

Glu Glu Glu Glu Gly Gly Cys Glu Leu Arg Val Lys Phe Ser Arg Ser
385                 390                 395                 400

Ala Asp Ala Pro Ala Tyr Gln Gln Gly Gln Asn Gln Leu Tyr Asn Glu
                405                 410                 415

Leu Asn Leu Gly Arg Arg Glu Glu Tyr Asp Val Leu Asp Lys Arg Arg
            420                 425                 430

Gly Arg Asp Pro Glu Met Gly Gly Lys Pro Arg Arg Lys Asn Pro Gln
        435                 440                 445

Glu Gly Leu Tyr Asn Glu Leu Gln Lys Asp Lys Met Ala Glu Ala Tyr
    450                 455                 460

Ser Glu Ile Gly Met Lys Gly Glu Arg Arg Arg Gly Lys Gly His Asp
465                 470                 475                 480

Gly Leu Tyr Gln Gly Leu Ser Thr Ala Thr Lys Asp Thr Tyr Asp Ala
                485                 490                 495

Leu His Met Gln Ala Leu Pro Pro Arg
            500                 505

<210>  6
<211>  294
<212>  PRT
<213>  Artificial Sequence

<220>
<223>  P4G2CAR

<400>  6

Met Ala Leu Pro Val Thr Ala Leu Leu Leu Pro Leu Ala Leu Leu Leu
1               5                   10                  15

His Ala Ala Arg Pro Gly Ser Gln Val Gln Leu Gln Gln Ser Gly Pro
            20                  25                  30

Gly Leu Val Thr Pro Ser Gln Thr Leu Ser Leu Thr Cys Ala Ile Ser
        35                  40                  45

Gly Asp Ser Val Ser Ser Asn Ser Ala Thr Trp Asn Trp Ile Arg Gln
    50                  55                  60

Ser Pro Ser Arg Gly Leu Glu Trp Leu Gly Arg Thr Tyr Tyr Arg Ser
65                  70                  75                  80

Lys Trp Tyr Asn Asp Tyr Ala Val Ser Val Lys Ser Arg Met Ser Ile
                85                  90                  95

Asn Pro Asp Thr Ser Lys Asn Gln Phe Ser Leu Gln Leu Asn Ser Val

100                 105                 110

Thr Pro Glu Asp Thr Ala Val Tyr Tyr Cys Ala Arg Gly Met Met Thr
        115                 120                 125

Tyr Tyr Tyr Gly Met Asp Val Trp Gly Gln Gly Thr Thr Val Thr Val
    130                 135                 140

Ser Ser Gly Ile Leu Gly Ser Gly Gly Gly Gly Ser Gly Gly Gly Gly
145                 150                 155                 160

Ser Gly Gly Gly Gly Ser Gln Pro Val Leu Thr Gln Ser Ser Ser Leu
                165                 170                 175

Ser Ala Ser Pro Gly Ala Ser Ala Ser Leu Thr Cys Thr Leu Arg Ser
            180                 185                 190

Gly Ile Asn Val Gly Pro Tyr Arg Ile Tyr Trp Tyr Gln Gln Lys Pro
        195                 200                 205

Gly Ser Pro Pro Gln Tyr Leu Leu Asn Tyr Lys Ser Asp Ser Asp Lys
    210                 215                 220

Gln Gln Gly Ser Gly Val Pro Ser Arg Phe Ser Gly Ser Lys Asp Ala
225                 230                 235                 240

Ser Ala Asn Ala Gly Val Leu Leu Ile Ser Gly Leu Arg Ser Glu Asp
                245                 250                 255

Glu Ala Asp Tyr Tyr Cys Met Ile Trp His Ser Ser Ala Ala Val Phe
            260                 265                 270

Gly Gly Gly Thr Gln Leu Thr Val Leu Ser Ala Ser Phe Val Pro Val
        275                 280                 285

Phe Leu Pro Ala Lys Pro
    290

<210>  7
<211>  3282
<212>  DNA
<213>  Artificial Sequence

<220>
<223>  GC33CAR-IRES-S1PR1

<400>  7
atggccttac cagtgaccgc cttgctcctg ccgctggcct tgctgctcca cgccgccagg      60

ccggacgtgg tgatgaccca gagcccttta tctttacccg ttacacccgg tgagcccgct     120

agcatctctt gtagaagcag ccagtcttta gtgcacagca acggcaacac atatttacac 180

tggtatttac agaagcccgg tcagagcccc cagctgctga tctacaaggt gagcaatcgt 240

ttctccggcg tgcccgatag attcagcggc agcggctccg gaaccgactt cactttaaag 300

atcagcagag tggaggccga ggacgtgggt gtgtactact gctcccagaa cacccacgtg 360

ccccctacat tcggtcaagg taccaaactg gagatcaaag gtggcggtgg ctcgggcggt 420

ggtgggtcgg gtggcggcgg atctcaagtt cagctggtgc agagcggcgc cgaggtcaaa 480

aaacccggag ccagcgtgaa ggtgtcttgt aaagccagcg gctacacctt taccgactat 540

gagatgcact gggtgagaca agctcccggt caaggtctgg aatggatggg cgctttagac 600

cccaagactg gtgacacagc ctactcccag aagttcaagg gtcgtgtgac tttaacagcc 660

gacgagtcca ccagcacagc ctacatggaa ctgagctctt taaggagcga ggacaccgcc 720

gtgtattact gcactcgttt ctacagctac acctactggg gccaaggtac tttagtgaca 780

gtgagcagct tcgtgccggt cttcctgcca gcgaagccca ccacgacgcc agcgccgcga 840

ccaccaacac cggcgcccac catcgcgtcg cagcccctgt ccctgcgccc agaggcgtgc 900

cggccagcgg cggggggcgc agtgcacacg agggggctgg acttcgcctg tgatatctac 960

atctgggcgc ccttggccgg gacttgtggg gtccttctcc tgtcactggt tatcaccctt 1020

tactgcaacc acaggaaccg tttctctgtt gttaaacggg gcagaaagaa gctcctgtat 1080

atattcaaac aaccatttat gagaccagta caaactactc aagaggaaga tggctgtagc 1140

tgccgatttc cagaagaaga agaaggagga tgtgaactga gagtgaagtt cagcaggagc 1200

gcagacgccc ccgcgtacca gcagggccag aaccagctct ataacgagct caatctagga 1260

cgaagagagg agtacgatgt tttggacaaa agaagaggcc gggaccctga gatgggggga 1320

aagccgagaa ggaagaaccc tcaggaaggc ctgtacaatg aactgcagaa agataagatg 1380

gcggaggcct acagtgagat tgggatgaaa ggcgagcgcc ggaggggcaa ggggcacgat 1440

ggcctttacc agggtctcag tacagccacc aaggacacct acgacgccct tcacatgcag 1500

gccctgcccc ctcgctaagg atcccgcccc tctccctccc ccccccctaa cgttactggc 1560

cgaagccgct tggaataagg ccggtgtgcg tttgtctata tgttattttc caccatattg 1620

ccgtcttttg gcaatgtgag ggcccggaaa cctggccctg tcttcttgac gagcattcct 1680

aggggtcttt cccctctcgc caaaggaatg caaggtctgt tgaatgtcgt gaaggaagca 1740

gttcctctgg aagcttcttg aagacaaaca acgtctgtag cgaccctttg caggcagcgg 1800

aaccccccac ctggcgacag gtgcctctgc ggccaaaagc cacgtgtata agatacacct 1860

gcaaaggcgg cacaacccca gtgccacgtt gtgagttgga tagttgtgga aagagtcaaa 1920

tggctctcct caagcgtatt caacaagggg ctgaaggatg cccagaaggt accccattgt 1980

atgggatctg atctggggcc tcggtgcaca tgctttacat gtgtttagtc gaggttaaaa     2040

aaacgtctag gccccccgaa ccacggggac gtggttttcc tttgaaaaac acgatgataa     2100

gcttgccaca acccacaatc tagaatgggg cccaccagcg tcccgctggt caaggcccac     2160

cgcagctcgg tctctgacta cgtcaactat gatatcatcg tccggcatta caactacacg     2220

ggaaagctga atatcagcgc ggacaaggag aacagcatta aactgacctc ggtggtgttc     2280

attctcatct gctgctttat catcctggag aacatctttg tcttgctgac catttggaaa     2340

accaagaaat tccaccgacc catgtactat tttattggca atctggccct ctcagacctg     2400

ttggcaggag tagcctacac agctaacctg ctcttgtctg gggccaccac ctacaagctc     2460

actcccgccc agtggtttct gcgggaaggg agtatgtttg tggccctgtc agcctccgtg     2520

ttcagtctcc tcgccatcgc cattgagcgc tatatcacaa tgctgaaaat gaaactccac     2580

aacgggagca ataacttccg cctcttcctg ctaatcagcg cctgctgggt catctccctc     2640

atcctgggtg gcctgcctat catgggctgg aactgcatca gtgcgctgtc cagctgctcc     2700

accgtgctgc cgctctacca caagcactat atcctcttct gcaccacggt cttcactctg     2760

cttctgctct ccatcgtcat tctgtactgc agaatctact ccttggtcag gactcggagc     2820

cgccgcctga cgttccgcaa gaacatttcc aaggccagcc gcagctctga gaagtcgctg     2880

gcgctgctca agaccgtaat tatcgtcctg agcgtcttca tcgcctgctg ggcaccgctc     2940

ttcatcctgc tcctgctgga tgtgggctgc aaggtgaaga cctgtgacat cctcttcaga     3000

gcggagtact tcctggtgtt agctgtgctc aactccggca ccaaccccat catttacact     3060

ctgaccaaca aggagatgcg tcgggccttc atccggatca tgtcctgctg caagtgcccg     3120

agcggagact ctgctggcaa attcaagcga cccatcatcg ccggcatgga attcagccgc     3180

agcaaatcgg acaattcctc ccacccccag aaagacgaag gggacaaccc agagaccatt     3240

atgtcttctg gaaacgtcaa ctcttcttcc ggaagcggat aa                        3282

<210>  8
<211>  2727
<212>  DNA
<213>  Artificial Sequence

<220>
<223>  S1PR1-2A-GC33CAR

<400>  8
atggggccca ccagcgtccc gctggtcaag gcccaccgca gctcggtctc tgactacgtc       60

aactatgata tcatcgtccg gcattacaac tacacgggaa agctgaatat cagcgcggac      120

aaggagaaca gcattaaact gacctcggtg gtgttcattc tcatctgctg ctttatcatc      180

ctggagaaca tctttgtctt gctgaccatt tggaaaacca agaaattcca ccgacccatg      240

tactatttta ttggcaatct ggccctctca gacctgttgg caggagtagc ctacacagct      300

aacctgctct tgtctggggc caccacctac aagctcactc ccgcccagtg gtttctgcgg 360

gaagggagta tgtttgtggc cctgtcagcc tccgtgttca gtctcctcgc catcgccatt 420

gagcgctata tcacaatgct gaaaatgaaa ctccacaacg ggagcaataa cttccgcctc 480

ttcctgctaa tcagcgcctg ctgggtcatc tccctcatcc tgggtggcct gcctatcatg 540

ggctggaact gcatcagtgc gctgtccagc tgctccaccg tgctgccgct ctaccacaag 600

cactatatcc tcttctgcac cacggtcttc actctgcttc tgctctccat cgtcattctg 660

tactgcagaa tctactcctt ggtcaggact cggagccgcc gcctgacgtt ccgcaagaac 720

atttccaagg ccagccgcag ctctgagaag tcgctggcgc tgctcaagac cgtaattatc 780

gtcctgagcg tcttcatcgc ctgctgggca ccgctcttca tcctgctcct gctggatgtg 840

ggctgcaagg tgaagacctg tgacatcctc ttcagagcgg agtacttcct ggtgttagct 900

gtgctcaact ccggcaccaa ccccatcatt tacactctga ccaacaagga gatgcgtcgg 960

gccttcatcc ggatcatgtc ctgctgcaag tgcccgagcg gagactctgc tggcaaattc 1020

aagcgaccca tcatcgccgg catggaattc agccgcagca aatcggacaa ttcctcccac 1080

ccccagaaag acgaagggga caacccagag accattatgt cttctggaaa cgtcaactct 1140

tcttccggaa gcggaggaag cggagagggc agaggaagtc tgctaacatg cggtgacgtc 1200

gaggagaatc ctggacctat ggccttacca gtgaccgcct tgctcctgcc gctggccttg 1260

ctgctccacg ccgccaggcc ggacgtggtg atgacccaga gccctttatc tttacccgtt 1320

acacccggtg agcccgctag catctcttgt agaagcagcc agtctttagt gcacagcaac 1380

ggcaacacat atttacactg gtatttacag aagcccggtc agagccccca gctgctgatc 1440

tacaaggtga gcaatcgttt ctccggcgtg cccgatagat tcagcggcag cggctccgga 1500

accgacttca ctttaaagat cagcagagtg gaggccgagg acgtgggtgt gtactactgc 1560

tcccagaaca cccacgtgcc ccctacattc ggtcaaggta ccaaactgga gatcaaaggt 1620

ggcggtggct cgggcggtgg tgggtcgggt ggcggcggat ctcaagttca gctggtgcag 1680

agcggcgccg aggtcaaaaa acccggagcc agcgtgaagg tgtcttgtaa agccagcggc 1740

tacaccttta ccgactatga gatgcactgg gtgagacaag ctcccggtca aggtctggaa 1800

tggatgggcg ctttagaccc caagactggt gacacagcct actcccagaa gttcaagggt 1860

cgtgtgactt taacagccga cgagtccacc agcacagcct acatggaact gagctcttta 1920

aggagcgagg acaccgccgt gtattactgc actcgtttct acagctacac ctactggggc 1980

caaggtactt tagtgacagt gagcagcttc gtgccggtct tcctgccagc gaagcccacc 2040

acgacgccag cgccgcgacc accaacaccg gcgcccacca tcgcgtcgca gcccctgtcc 2100

ctgcgcccag aggcgtgccg gccagcggcg gggggcgcag tgcacacgag ggggctggac 2160

ttcgcctgtg atttctggtt acccatagga tgtgcagcct ttgttgtagt ctgcattttg 2220

ggatgcatac ttatttgttg gcttacacgt ttctctgttg ttaaacgggg cagaaagaag 2280

ctcctgtata tattcaaaca accatttatg agaccagtac aaactactca agaggaagat 2340

ggctgtagct gccgatttcc agaagaagaa gaaggaggat gtgaactgag agtgaagttc 2400

agcaggagcg cagacgcccc cgcgtaccag cagggccaga accagctcta taacgagctc 2460

aatctaggac gaagagagga gtacgatgtt ttggacaaga gacgtggccg ggaccctgag 2520

atggggggaa agccgagaag gaagaaccct caggaaggcc tgtacaatga actgcagaaa 2580

gataagatgg cggaggccta cagtgagatt gggatgaaag gcgagcgccg gaggggcaag 2640

gggcacgatg gcctttacca gggtctcagt acagccacca aggacaccta cgacgccctt 2700

cacatgcagg ccctgccccc tcgctaa 2727

<210> 9
<211> 2799
<212> DNA
<213> Artificial Sequence

<220>
<223> S1PR1-2A-P4G2CAR

<400> 9
atggggccca ccagcgtccc gctggtcaag gcccaccgca gctcggtctc tgactacgtc 60

aactatgata tcatcgtccg gcattacaac tacacgggaa agctgaatat cagcgcggac 120

aaggagaaca gcattaaact gacctcggtg gtgttcattc tcatctgctg ctttatcatc 180

ctggagaaca tctttgtctt gctgaccatt tggaaaacca agaaattcca ccgacccatg 240

tactatttta ttggcaatct ggccctctca gacctgttgg caggagtagc ctacacagct 300

aacctgctct tgtctggggc caccacctac aagctcactc ccgcccagtg gtttctgcgg 360

gaagggagta tgtttgtggc cctgtcagcc tccgtgttca gtctcctcgc catcgccatt 420

gagcgctata tcacaatgct gaaaatgaaa ctccacaacg ggagcaataa cttccgcctc 480

ttcctgctaa tcagcgcctg ctgggtcatc tccctcatcc tgggtggcct gcctatcatg 540

ggctggaact gcatcagtgc gctgtccagc tgctccaccg tgctgccgct ctaccacaag 600

cactatatcc tcttctgcac cacggtcttc actctgcttc tgctctccat cgtcattctg 660

tactgcagaa tctactcctt ggtcaggact cggagccgcc gcctgacgtt ccgcaagaac 720

atttccaagg ccagccgcag ctctgagaag tcgctggcgc tgctcaagac cgtaattatc 780

gtcctgagcg tcttcatcgc ctgctgggca ccgctcttca tcctgctcct gctggatgtg 840

ggctgcaagg tgaagacctg tgacatcctc ttcagagcgg agtacttcct ggtgttagct 900

gtgctcaact ccggcaccaa ccccatcatt tacactctga ccaacaagga gatgcgtcgg 960

gccttcatcc ggatcatgtc ctgctgcaag tgcccgagcg gagactctgc tggcaaattc 1020

aagcgaccca tcatcgccgg catggaattc agccgcagca aatcggacaa ttcctcccac 1080

ccccagaaag acgaagggga caacccagag accattatgt cttctggaaa cgtcaactct 1140

tcttccggaa gcggaggaag cggagagggc agaggaagtc tgctaacatg cggtgacgtc 1200

gaggagaatc ctggacctat ggctctgccc gtgaccgctc tgctgctgcc tctcgctctg 1260

ctgctgcatg ccgccagacc cggctcccaa gtgcaactcc agcaatccgg acccggactg 1320

gtgacaccct cccaaacact gtccctcaca tgcgccatca gcggagattc cgtgtcctcc 1380

aacagcgcta catggaattg gattagacag agcccttcca gaggactgga gtggctcgga 1440

agaacatact acagatccaa gtggtataac gactacgccg tgtccgtgaa gtctagaatg 1500

agcatcaacc ccgacaccag caagaaccag ttttctctgc agctgaacag cgtgacaccc 1560

gaggacaccg ctgtgtacta ctgcgccaga ggcatgatga cctactacta cggcatggac 1620

gtctggggac aaggcaccac cgtgacagtg agcagcggca ttctgggctc cggaggaggc 1680

ggaagcggag gcggaggcag cggaggcggc ggaagccaac ccgtgctcac acaaagctcc 1740

tctctgagcg ctagccccgg cgcctccgct tctctgacat gtacactgag gagcggcatc 1800

aacgtcggac cctatagaat ctactggtac cagcagaagc ccggcagccc cccccaatat 1860

ctgctgaact acaagtccga ctccgacaag cagcaaggca gcggcgtgcc ttctagattc 1920

agcggcagca aggacgctag cgccaacgcc ggagtgctgc tgatctccgg actgagaagc 1980

gaggacgagg ccgactacta ttgcatgatc tggcactcca gcgccgctgt ctttggagga 2040

ggcacacagc tgacagtgct gagcgccagc ttcgtgcccg tgttcctccc cgccaaaccc 2100

accacaacac ccgcccctag accccctaca cccgccccta ccatcgcttc ccagcctctg 2160

tctctgagac ccgaggcttg tagacccgcc gctggaggag ctgtgcatac aagaggactg 2220

gacttcgctt gcgacatcta tatttgggct cccctcgctg gaacatgcgg agtgctgctg 2280

ctgtctctgg tgatcacact gtactgcaac catagaaaca gattctccgt ggtgaagaga 2340

ggaagaaaga aactgctcta catcttcaag cagcccttca tgaggcccgt gcagaccaca 2400

caagaggagg acggatgtag ctgtagattc cccgaagagg aggagggagg atgtgagctg 2460

agagtgaagt tctctagatc cgccgatgcc cccgcctacc agcaaggcca gaaccagctc 2520

tataacgagc tcaatctggg aagaagagag gagtacgacg tgctggacaa gaggagggga 2580

agagaccccg aaatgggcgg caagcccaga aggaaaaacc cccaagaggg actgtacaac 2640

gaactgcaga aggacaagat ggctgaggcc tacagcgaaa tcggcatgaa aggcgagagg 2700

aggagaggca aaggacacga cggactctat caaggcctca gcaccgccac caaggacacc 2760

tacgatgctc tgcatatgca agctctgccc cctagataa 2799

<210> 10

<211>  2484
<212>  DNA
<213>  Artificial Sequence

<220>
<223>  S1PR1-IRES-GFP

<400>  10
atggggccca ccagcgtccc gctggtcaag gcccaccgca gctcggtctc tgactacgtc      60

aactatgata tcatcgtccg gcattacaac tacacgggaa agctgaatat cagcgcggac     120

aaggagaaca gcattaaact gacctcggtg gtgttcattc tcatctgctg ctttatcatc     180

ctggagaaca tctttgtctt gctgaccatt tggaaaacca agaaattcca ccgacccatg     240

tactatttta ttggcaatct ggccctctca gacctgttgg caggagtagc ctacacagct     300

aacctgctct tgtctggggc caccacctac aagctcactc ccgcccagtg gtttctgcgg     360

gaagggagta tgtttgtggc cctgtcagcc tccgtgttca gtctcctcgc catcgccatt     420

gagcgctata tcacaatgct gaaaatgaaa ctccacaacg ggagcaataa cttccgcctc     480

ttcctgctaa tcagcgcctg ctgggtcatc tccctcatcc tgggtggcct gcctatcatg     540

ggctggaact gcatcagtgc gctgtccagc tgctccaccg tgctgccgct ctaccacaag     600

cactatatcc tcttctgcac cacggtcttc actctgcttc tgctctccat cgtcattctg     660

tactgcagaa tctactcctt ggtcaggact cggagccgcc gcctgacgtt ccgcaagaac     720

atttccaagg ccagccgcag ctctgagaag tcgctggcgc tgctcaagac cgtaattatc     780

gtcctgagcg tcttcatcgc ctgctgggca ccgctcttca tcctgctcct gctggatgtg     840

ggctgcaagg tgaagacctg tgacatcctc ttcagagcgg agtacttcct ggtgttagct     900

gtgctcaact ccggcaccaa ccccatcatt tacactctga ccaacaagga gatgcgtcgg     960

gccttcatcc ggatcatgtc ctgctgcaag tgcccgagcg gagactctgc tggcaaattc    1020

aagcgaccca tcatcgccgg catggaattc agccgcagca aatcggacaa ttcctcccac    1080

ccccagaaag acgaagggga caacccagag accattatgt cttctggaaa cgtcaactct    1140

tcttccggaa gcggataagg atcccgcccc tctccctccc ccccccctaa cgttactggc    1200

cgaagccgct tggaataagg ccggtgtgcg tttgtctata tgttattttc caccatattg    1260

ccgtcttttg gcaatgtgag ggcccggaaa cctggccctg tcttcttgac gagcattcct    1320

aggggtcttt cccctctcgc caaaggaatg caaggtctgt tgaatgtcgt gaaggaagca    1380

gttcctctgg aagcttcttg aagacaaaca acgtctgtag cgaccctttg caggcagcgg    1440

aaccccccac ctggcgacag gtgcctctgc ggccaaaagc cacgtgtata agatacacct    1500

gcaaaggcgg cacaacccca gtgccacgtt gtgagttgga tagttgtgga aagagtcaaa    1560

tggctctcct caagcgtatt caacaagggg ctgaaggatg cccagaaggt accccattgt    1620

atgggatctg atctggggcc tcggtgcaca tgctttacat gtgtttagtc gaggttaaaa    1680

aaacgtctag gccccccgaa ccacggggac gtggttttcc tttgaaaaac acgatgataa 1740

gcttgccaca acccacaatc tagaatggtg agcaagggcg aggagctgtt caccggggtg 1800

gtgcccatcc tggtcgagct ggacggcgac gtaaacggcc acaagttcag cgtgtccggc 1860

gagggcgagg gcgatgccac ctacggcaag ctgaccctga agttcatctg caccaccggc 1920

aagctgcccg tgccctggcc caccctcgtg accaccctga cctacggcgt gcagtgcttc 1980

agccgctacc ccgaccacat gaagcagcac gacttcttca agtccgccat gcccgaaggc 2040

tacgtccagg agcgcaccat cttcttcaag gacgacggca actacaagac ccgcgccgag 2100

gtgaagttcg agggcgacac cctggtgaac cgcatcgagc tgaagggcat cgacttcaag 2160

gaggacggca acatcctggg gcacaagctg gagtacaact acaacagcca caacgtctat 2220

atcatggccg acaagcagaa gaacggcatc aaggtgaact tcaagatccg ccacaacatc 2280

gaggacggca gcgtgcagct cgccgaccac taccagcaga acacccccat cggcgacggc 2340

cccgtgctgc tgcccgacaa ccactacctg agcacccagt ccgccctgag caaagacccc 2400

aacgagaagc gcgatcacat ggtcctgctg gagttcgtga ccgccgccgg gatcactctc 2460

ggcatggacg agctgtacaa gtaa 2484

<210> 11
<211> 2844
<212> DNA
<213> Artificial Sequence

<220>
<223> GC33CAR-IRES-GFP

<400> 11
atggccttac cagtgaccgc cttgctcctg ccgctggcct tgctgctcca cgccgccagg 60

ccggacgtgg tgatgaccca gagcccttta tctttacccg ttacacccgg tgagcccgct 120

agcatctctt gtagaagcag ccagtcttta gtgcacagca acggcaacac atatttacac 180

tggtatttac agaagcccgg tcagagcccc cagctgctga tctacaaggt gagcaatcgt 240

ttctccggcg tgcccgatag attcagcggc agcggctccg gaaccgactt cactttaaag 300

atcagcagag tggaggccga ggacgtgggt gtgtactact gctcccagaa cacccacgtg 360

ccccctacat tcggtcaagg taccaaactg gagatcaaag gtggcggtgg ctcgggcggt 420

ggtgggtcgg gtggcggcgg atctcaagtt cagctggtgc agagcggcgc cgaggtcaaa 480

aaacccggag ccagcgtgaa ggtgtcttgt aaagccagcg gctacacctt taccgactat 540

gagatgcact gggtgagaca agctcccggt caaggtctgg aatggatggg cgctttagac 600

cccaagactg gtgacacagc ctactcccag aagttcaagg gtcgtgtgac tttaacagcc 660

gacgagtcca ccagcacagc ctacatggaa ctgagctctt taaggagcga ggacaccgcc 720

gtgtattact gcactcgttt ctacagctac acctactggg gccaaggtac tttagtgaca 780

gtgagcagct tcgtgccggt cttcctgcca gcgaagccca ccacgacgcc agcgccgcga 840

ccaccaacac cggcgcccac catcgcgtcg cagcccctgt ccctgcgccc agaggcgtgc 900

cggccagcgg cggggggcgc agtgcacacg agggggctgg acttcgcctg tgatatctac 960

atctgggcgc ccttggccgg gacttgtggg gtccttctcc tgtcactggt tatcaccctt 1020

tactgcaacc acaggaaccg tttctctgtt gttaaacggg gcagaaagaa gctcctgtat 1080

atattcaaac aaccatttat gagaccagta caaactactc aagaggaaga tggctgtagc 1140

tgccgatttc cagaagaaga agaaggagga tgtgaactga gagtgaagtt cagcaggagc 1200

gcagacgccc ccgcgtacca gcagggccag aaccagctct ataacgagct caatctagga 1260

cgaagagagg agtacgatgt tttggacaaa agaagaggcc gggaccctga gatgggggga 1320

aagccgagaa ggaagaaccc tcaggaaggc ctgtacaatg aactgcagaa agataagatg 1380

gcggaggcct acagtgagat tgggatgaaa ggcgagcgcc ggaggggcaa ggggcacgat 1440

ggcctttacc agggtctcag tacagccacc aaggacacct acgacgccct tcacatgcag 1500

gccctgcccc ctcgctaagg atcccgcccc tctccctccc ccccccctaa cgttactggc 1560

cgaagccgct tggaataagg ccggtgtgcg tttgtctata tgttattttc caccatattg 1620

ccgtcttttg gcaatgtgag ggcccggaaa cctggccctg tcttcttgac gagcattcct 1680

aggggtcttt cccctctcgc caaaggaatg caaggtctgt tgaatgtcgt gaaggaagca 1740

gttcctctgg aagcttcttg aagacaaaca acgtctgtag cgaccctttg caggcagcgg 1800

aaccccccac ctggcgacag gtgcctctgc ggccaaaagc cacgtgtata agatacacct 1860

gcaaaggcgg cacaacccca gtgccacgtt gtgagttgga tagttgtgga aagagtcaaa 1920

tggctctcct caagcgtatt caacaagggg ctgaaggatg cccagaaggt accccattgt 1980

atgggatctg atctggggcc tcggtgcaca tgctttacat gtgtttagtc gaggttaaaa 2040

aaacgtctag gccccccgaa ccacggggac gtggttttcc tttgaaaaac acgatgataa 2100

gcttgccaca acccacaatc tagaatggtg agcaagggcg aggagctgtt caccggggtg 2160

gtgcccatcc tggtcgagct ggacggcgac gtaaacggcc acaagttcag cgtgtccggc 2220

gagggcgagg gcgatgccac ctacggcaag ctgaccctga agttcatctg caccaccggc 2280

aagctgcccg tgccctggcc caccctcgtg accaccctga cctacggcgt gcagtgcttc 2340

agccgctacc ccgaccacat gaagcagcac gacttcttca agtccgccat gcccgaaggc 2400

tacgtccagg agcgcaccat cttcttcaag gacgacggca actacaagac ccgcgccgag 2460

gtgaagttcg agggcgacac cctggtgaac cgcatcgagc tgaagggcat cgacttcaag 2520

gaggacggca acatcctggg gcacaagctg gagtacaact acaacagcca caacgtctat 2580

atcatggccg acaagcagaa gaacggcatc aaggtgaact tcaagatccg ccacaacatc 2640

gaggacggca gcgtgcagct cgccgaccac taccagcaga acacccccat cggcgacggc 2700

cccgtgctgc tgcccgacaa ccactacctg agcacccagt ccgccctgag caaagacccc 2760

aacgagaagc gcgatcacat ggtcctgctg gagttcgtga ccgccgccgg gatcactctc 2820

ggcatggacg agctgtacaa gtaa 2844

<210> 12
<211> 3282
<212> DNA
<213> Artificial Sequence

<220>
<223> S1PR1-IRES-GC33CAR

<400> 12
atgggggcca ccagcgtccc gctggtcaag gcccaccgca gctcggtctc tgactacgtc 60

aactatgata tcatcgtccg gcattacaac tacacgggaa agctgaatat cagcgcggac 120

aaggagaaca gcattaaact gacctcggtg gtgttcattc tcatctgctg ctttatcatc 180

ctggagaaca tctttgtctt gctgaccatt tggaaaacca agaaattcca ccgacccatg 240

tactatttta ttggcaatct ggccctctca gacctgttgg caggagtagc ctacacagct 300

aacctgctct tgtctggggc caccacctac aagctcactc ccgcccagtg gtttctgcgg 360

gaagggagta tgtttgtggc cctgtcagcc tccgtgttca gtctcctcgc catcgccatt 420

gagcgctata tcacaatgct gaaaatgaaa ctccacaacg ggagcaataa cttccgcctc 480

ttcctgctaa tcagcgcctg ctgggtcatc tccctcatcc tgggtggcct gcctatcatg 540

ggctggaact gcatcagtgc gctgtccagc tgctccaccg tgctgccgct ctaccacaag 600

cactatatcc tcttctgcac cacggtcttc actctgcttc tgctctccat cgtcattctg 660

tactgcagaa tctactcctt ggtcaggact cggagccgcc gcctgacgtt ccgcaagaac 720

atttccaagg ccagccgcag ctctgagaag tcgctggcgc tgctcaagac cgtaattatc 780

gtcctgagcg tcttcatcgc ctgctgggca ccgctcttca tcctgctcct gctggatgtg 840

ggctgcaagg tgaagacctg tgacatcctc ttcagagcgg agtacttcct ggtgttagct 900

gtgctcaact ccggcaccaa ccccatcatt tacactctga ccaacaagga gatgcgtcgg 960

gccttcatcc ggatcatgtc ctgctgcaag tgcccgagcg gagactctgc tggcaaattc 1020

aagcgaccca tcatcgccgg catggaattc agccgcagca aatcggacaa ttcctcccac 1080

ccccagaaag acgaagggga caacccagag accattatgt cttctggaaa cgtcaactct 1140

tcttccggaa gcggataagg atcccgcccc tctccctccc ccccccctaa cgttactggc 1200

cgaagccgct tggaataagg ccggtgtgcg tttgtctata tgttattttc caccatattg 1260

ccgtcttttg gcaatgtgag ggcccggaaa cctggccctg tcttcttgac gagcattcct 1320

aggggtcttt cccctctcgc caaaggaatg caaggtctgt tgaatgtcgt gaaggaagca 1380

gttcctctgg aagcttcttg aagacaaaca acgtctgtag cgaccctttg caggcagcgg 1440

aaccccccac ctggcgacag gtgcctctgc ggccaaaagc cacgtgtata agatacacct 1500

gcaaaggcgg cacaacccca gtgccacgtt gtgagttgga tagttgtgga aagagtcaaa 1560

tggctctcct caagcgtatt caacaagggg ctgaaggatg cccagaaggt accccattgt 1620

atgggatctg atctggggcc tcggtgcaca tgctttacat gtgtttagtc gaggttaaaa 1680

aaacgtctag gccccccgaa ccacggggac gtggttttcc tttgaaaaac acgatgataa 1740

gcttgccaca acccacaatc tagaatggcc ttaccagtga ccgccttgct cctgccgctg 1800

gccttgctgc tccacgccgc caggccggac gtggtgatga cccagagccc tttatcttta 1860

cccgttacac ccggtgagcc cgctagcatc tcttgtagaa gcagccagtc tttagtgcac 1920

agcaacggca acacatattt acactggtat ttacagaagc ccggtcagag cccccagctg 1980

ctgatctaca aggtgagcaa tcgtttctcc ggcgtgcccg atagattcag cggcagcggc 2040

tccggaaccg acttcacttt aaagatcagc agagtggagg ccgaggacgt gggtgtgtac 2100

tactgctccc agaacaccca cgtgccccct acattcggtc aaggtaccaa actggagatc 2160

aaaggtggcg gtggctcggg cggtggtggg tcgggtggcg gcggatctca agttcagctg 2220

gtgcagagcg gcgccgaggt caaaaaaccc ggagccagcg tgaaggtgtc ttgtaaagcc 2280

agcggctaca cctttaccga ctatgagatg cactgggtga gacaagctcc cggtcaaggt 2340

ctggaatgga tgggcgcttt agaccccaag actggtgaca cagcctactc ccagaagttc 2400

aagggtcgtg tgactttaac agccgacgag tccaccagca cagcctacat ggaactgagc 2460

tctttaagga gcgaggacac cgccgtgtat tactgcactc gtttctacag ctacacctac 2520

tggggccaag gtactttagt gacagtgagc agcttcgtgc cggtcttcct gccagcgaag 2580

cccaccacga cgccagcgcc gcgaccacca acaccggcgc ccaccatcgc gtcgcagccc 2640

ctgtccctgc gcccagaggc gtgccggcca gcggcggggg gcgcagtgca cacgaggggg 2700

ctggacttcg cctgtgatat ctacatctgg gcgcccttgg ccgggacttg tggggtcctt 2760

ctcctgtcac tggttatcac cctttactgc aaccacagga accgtttctc tgttgttaaa 2820

cggggcagaa agaagctcct gtatatattc aaacaaccat ttatgagacc agtacaaact 2880

actcaagagg aagatggctg tagctgccga tttccagaag aagaagaagg aggatgtgaa 2940

ctgagagtga agttcagcag gagcgcagac gcccccgcgt accagcaggg ccagaaccag 3000

ctctataacg agctcaatct aggacgaaga gaggagtacg atgttttgga caaaagaaga 3060

ggccgggacc ctgagatggg gggaaagccg agaaggaaga accctcagga aggcctgtac 3120

aatgaactgc agaaagataa gatggcggag gcctacagtg agattgggat gaaaggcgag 3180

cgccggaggg gcaaggggca cgatggcctt taccagggtc tcagtacagc caccaaggac 3240

acctacgacg cccttcacat gcaggccctg ccccctcgct aa                    3282

<210>  13
<211>  574
<212>  DNA
<213>  Artificial Sequence

<220>
<223>  IRES

<400>  13
cccctctccc tccccccccc ctaacgttac tggccgaagc cgcttggaat aaggccggtg      60

tgcgtttgtc tatatgttat tttccaccat attgccgtct tttggcaatg tgagggcccg     120

gaaacctggc cctgtcttct tgacgagcat tcctaggggt ctttcccctc tcgccaaagg     180

aatgcaaggt ctgttgaatg tcgtgaagga agcagttcct ctggaagctt cttgaagaca     240

aacaacgtct gtagcgaccc tttgcaggca gcggaacccc ccacctggcg acaggtgcct     300

ctgcggccaa aagccacgtg tataagatac acctgcaaag gcggcacaac cccagtgcca     360

cgttgtgagt tggatagttg tggaaagagt caaatggctc tcctcaagcg tattcaacaa     420

ggggctgaag gatgcccaga aggtacccca ttgtatggga tctgatctgg ggcctcggtg     480

cacatgcttt acatgtgttt agtcgaggtt aaaaaaacgt ctaggccccc cgaaccacgg     540

ggacgtggtt ttcctttgaa aaacacgatg ataa                                574

<210>  14
<211>  54
<212>  DNA
<213>  Artificial Sequence

<220>
<223>  2A

<400>  14
gagggcagag gaagtctgct aacatgcggt gacgtcgagg agaatcctgg acct           54

<210>  15
<211>  720
<212>  DNA
<213>  Artificial Sequence

<220>
<223>  GFP

<400>  15
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgaccta cggcgtgcag tgcttcagcc gctaccccga ccacatgaag     240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actctcggca tggacgagct gtacaagtaa 720